# EUROPEAN PATENT SPECIFICATION

(11) **EP 4 331 538 B1**
(45) Date of publication and mention of the grant of the patent: **17.06.2026**
(21) Application number: 23180097.0
(22) Date of filing: 19.06.2023
(51) Int. Cl.: A61F 2/30, A61F 2/42

(54) **MULTIAXIAL MODULAR STEMS**
MEHRACHSIGE MODULARE SCHÄFTE
TIGES MODULAIRES MULTIAXIALES

(30) Priority: 01.09.2022 US 202263374248 P; 08.06.2023 US 202318331470
(43) Date of publication of application: 06.03.2024
(73) Proprietor: Wright Medical Technology, Inc., Memphis, TN 38117 (US)
(72) Inventor: LOWERY, Gary W., Eads, 38028 (US); MOORE, Jesse G., Germantown, 38138 (US)
(74) Representative: Zimmermann & Partner Patentanwälte mbB

(56) References cited:
- EP-A1- 0 385 930
- US-A- 4 011 602
- US-A- 4 878 917
- US-A1- 2017 079 798

## Description

### FIELD

The present disclosure is related to orthopedics. More particularly, the disclosed systems and methods relate to multi-component prosthesis stems.

### BACKGROUND

Tibia stem components help engage implants where limited bone is available for total ankle arthroplasty. Pistoning or loosening of the implant can present as a long term complication. Thus, improved tibia stem components that can better engage with the tibia bone and improve immediate implant stability and reduce implant migration over time are desired.
Document EP 0 385 930 A1 relates to a shaft for a femoral prosthesis.
Document US 4,878,917 A relates to a modular assembly for a shaft prosthesis.
Document US 4,011,602 A relates to a porous expandable device for attachment to tissue.
Document US 2017/079798 A1 relates to a hip joint device.

### SUMMARY

Disclosed herein are implants designed to engage cancellous, and possibly cortical, tibia bone to improve immediate implant stability and reduce implant migration over long term.

The invention is defined in claim 1. Disclosed is an ankle prosthesis comprising: a tibia stem component comprising a leading end, a trailing end, and a longitudinal axis defined therethrough; and a tibia tray component configured to be attached to a prosthetic joint articulating surface, wherein the tibia tray component extends from the trailing end of the tibia stem component, wherein the tibia stem component is sized and configured to be disposed in an intramedullary canal formed in a tibia, wherein the tibia stem component comprises: one or more retractable members configured to be controllably movable from a retracted position and be extended outward and away from the longitudinal axis, wherein in the retracted position, the one or more retractable members are contained substantially within the tibia stem component and do not extend outward, wherein when the tibia stem component is disposed in the intramedullary canal and the one or more retractable members are extended outward, the one or more retractable members engage the intramedullary canal's surrounding bone and enhance anchoring the tibia stem within the intramedullary canal.

In some aspects, the third component may include a base nut, and the actuator may include a threaded stem that is configured to be engaged by the base nut.

In some aspects, the actuator may include a cam surface disposed adjacent to the threaded stem. The cam surface may be configured to engage an internal surface of the retractable member.

In some aspects, the first counter-torque feature may include a protrusion extending from a head portion of the actuator that is disposed adjacent to the cam surface. The protrusion may be configured to be received in a slit disposed adjacent to the retractable member.

In some aspects, a prosthesis may include a washer sized and configured to be received between the first component and the second component and to receive the threaded stem. The washer may be configured to deform in response to a pressure applied by the first component and the second component.

In some aspects, a prosthesis may include a securing member configured to secure the actuator within the internal cavity.

In some aspects, the securing member may include a split ring sized and configured to be disposed on the threaded stem of the actuator.

In some aspects, the trailing end of the second component may include a second counter-torque feature configured to engage the first component and to resist rotation of the second component when the actuator is engaged by the third component.

In some aspects, the second counter-torque feature includes surface texturing.

In some aspects, the second counter-torque feature may include a protrusion that is configured to be received in a corresponding recess defined by the first component.

In some aspects, the second counter-torque feature may include a flat surface formed on the trailing end of the second component that is configured to engage a corresponding surface on the first component.

In some aspects, the second counter-torque feature may include a recess that is configured to receive a protrusion extending from a surface of the first component.

In some aspects, the third component may include a threaded bolt, and wherein the actuator includes a body having a cam surface and a head portion, the body defining a threaded hole at one end that is configured to at least partially receive the threaded bolt.

In some aspects, the first counter-torque feature may include a protrusion extending from the head portion. The protrusion may be sized and configured to be received in a slit disposed adjacent to the retractable member. The threaded hole may be a blind hole.

In some aspects, the threaded hole may extend through the body of the actuator, the body defining a second hole that receives a pin having a length that is greater than a width of the body.

In some aspects, a tip of the retractable member may include a projection configured to retain the actuator within the internal cavity.

In some aspects, the actuator may be configured to move the retractable member from the first position to the second position and the first counter-torque feature may be configured to resist rotation of the stem component when the actuator is actuated.

In some aspects, the retractable member may include a projection configured to retain the actuator within the internal cavity.

In some aspects, the first counter-torque feature may include a surface texture at the trailing end of the stem component.

In some aspects, the first counter-torque feature may include a protrusion extending from the actuator and may be disposed within a slit defined by the stem component.

In some aspects, the first counter-torque feature may include a protrusion extending from the trailing end of the stem component.

In some aspects, the first counter-torque feature may include a recess defined by the trailing end of the stem component, the recess sized and configured to receive a projection that extends from a surface of another component of the prosthesis.

In some aspects, the first counter-torque feature may include a flat formed at the trailing end of the stem component. The flat may be configured to be engaged by a complementary surface of another component of the prosthesis.

In some aspects, the actuator may include a first portion, a second portion, and a third portion. The second portion may include a threaded shaft that extends through a hole defined by the trailing end of the stem. The third portion may be disposed between the first portion and the second portion and include a cam surface.

In some aspects, a securing member may be disposed along a length of the second portion and retain the actuator within the internal cavity.

In some aspects, the securing member may include a split ring.

In some aspects, a prosthesis may include a deformable washer disposed between the trailing end of the stem and the securing member.

In some aspects, the first portion of the actuator may include a projection that is received within a slit disposed adjacent to the retractable member.

In some aspects, the trailing end of the stem component may include a second counter-torque feature.

In some aspects, the second counter-torque feature may include a surface texture at the trailing end of the stem component.

In some aspects, the second counter-torque feature may include a protrusion extending from the actuator. The protrusion may be disposed within a slit defined by the stem component.

In some aspects, the second counter-torque feature may include a protrusion extending from the trailing end of the stem component.

In some aspects, the second counter-torque feature may include a recess defined by the trailing end of the stem component. The recess may be sized and configured to receive a projection that extends from a surface of another component of the prosthesis.

In some aspects, the second counter-torque feature may include a flat formed at the trailing end of the stem component. The flat may be configured to be engaged by a complementary surface of another component of the prosthesis.

In some aspects, the actuator may include a body having a first portion and a second portion. The second portion may taper from the first portion and provide a cam surface. The body may define a hole that is at least partially threaded at one end.

In some aspects, the first counter-torque feature may include a protrusion extending from the first portion. The protrusion may be received within a slit disposed adjacent to the retractable member.

In some aspects, the hole defined by the body of the actuator may extend through the body. The body may define a transverse hole in which a pin is disposed. The pin may have a length that is greater than a width of the body such that at least a portion of the pin is received within a slit disposed adjacent to the retractable member.

In some aspects, the first counter-torque feature may include a surface texture at the trailing end of the stem component.

In some aspects, the first counter-torque feature may include a protrusion extending from the trailing end of the stem component.

In some aspects, the first counter-torque feature may include a recess defined by the trailing end of the stem component. The recess may be sized and configured to receive a projection that extends from a surface of another component of the prosthesis.

In some aspects, the first counter-torque feature may include a flat formed at the trailing end of the stem component. The flat may be configured to be engaged by a complementary surface of another component of the prosthesis.

Further disclosed herein are tibia stem components of ankle replacement prosthesis that are designed to engage dense tibia bone to reduce implant migration over long term.

### BRIEF DESCRIPTION OF DRAWINGS

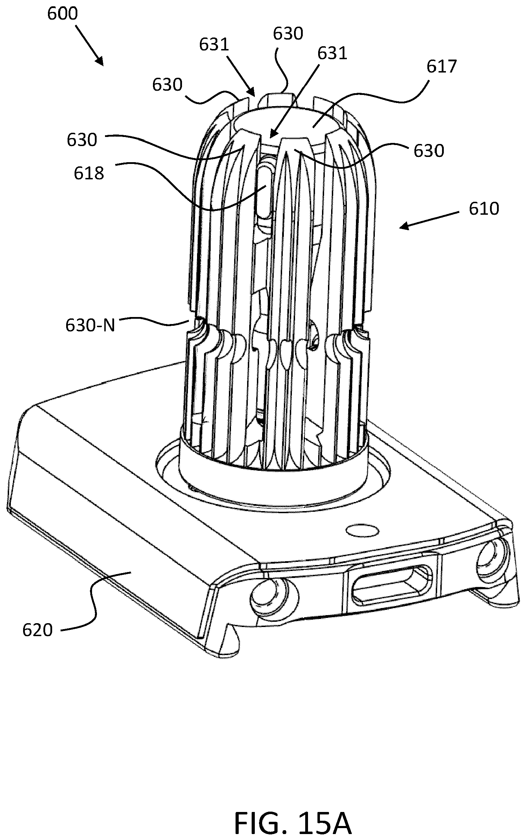

The features of the embodiments described herein will be more fully disclosed in the following detailed description, which is to be considered together with the accompanying drawings wherein like numbers refer to like parts. All drawings are schematic and are not intended to show actual dimensions or proportions. FIGS. 15A-15D, 17, 18A-18C, 19, 22 and 25-28. illustrate embodiments of the invention.
FIGS. 1A-1L are illustrations of a prosthesis according to an example of the present disclosure.
FIGS. 2A-2F are illustrations of a prosthesis according to another example of the present disclosure.
FIGS. 3A-3J are illustrations of a prosthesis according to another example of the present disclosure.
FIGS. 4A-4G are illustrations of a prosthesis according to another example of the present disclosure.
FIGS. 5A-5F are illustrations of a prosthesis according to another example of the present disclosure.
FIGS. 6A-6G are illustrations of a prosthesis according to another example of the present disclosure.
FIGS. 7A-7G are illustrations of a prosthesis according to another example of the present disclosure.
FIGS. 8A-8G are illustrations of a prosthesis according to another example of the present disclosure.
FIGS. 9A-9C are illustrations of a prosthesis according to another example of the present disclosure.
FIGS. 9D-9F are illustrations of a prosthesis according to another example of the present disclosure.
FIGS. 10A-10C are illustrations of a prosthesis according to another example of the present disclosure.
FIGS. 10D-10H are illustrations of a prosthesis according to another example of the present disclosure.
FIGS. 11A-11F are illustrations of a prosthesis according to another example of the present disclosure.
FIGS. 12A-12H are illustrations of a prosthesis according to another example of the present disclosure.
FIGS. 13A-13B are illustrations of a prosthesis according to another example of the present disclosure;
FIG. 14A is an isometric view of another example of a prosthesis with a counter-torque feature;
FIG. 14B is an exploded, top side, isometric view of the prosthesis illustrated in FIG. 14A;
FIG. 14C is an exploded, bottom side, isometric view of the prosthesis illustrated in FIG. 14A;
FIG. 15A is an isometric view of another example of a prosthesis with a counter-torque feature in accordance with embodiments of the invention;
FIG. 15B is an exploded, top side, isometric view of the prosthesis illustrated in FIG. 15A in accordance with some embodiments.
FIG. 15C is an exploded, bottom side, isometric view of the prosthesis illustrated in FIG. 15A in accordance with some embodiments;
FIG. 15D is a cross-sectional view of the prosthesis illustrated in FIG. 15A in accordance with some embodiments;
FIG. 16 is a schematic representation of a tibia stem component including distal ends that trap a linear actuator within a cavity defined by the tibia stem component;
FIG. 17 is an exploded, top side, isometric view of another example of an implant in accordance with embodiments of the invention;
FIG. 18A is an exploded, top side, isometric view of another example of an implant in accordance with some embodiments;
FIG. 18B is a front side view of the implant illustrated in FIG. 18A in accordance with some embodiments;
FIG. 18C is a sectional view of the implant illustrated in FIG. 18A taken along line A-A in FIG. 18B in accordance with some embodiments;
FIG. 19A is a cross-sectional view of another example of a modular prosthesis in accordance with some embodiments;
FIG. 19B is a detailed view of an interface between a counter-torque feature and a washer of the modular prosthesis illustrated in FIG. 19A in accordance with some embodiments;
FIG. 20 is a partial cross-sectional view of another example of a prosthesis;
FIG. 21 is a partial cross-sectional view of another example of a prosthesis;
FIG. 22 is a partial cross-sectional view of another example of a prosthesis in accordance with embodiments of the invention;
FIG. 23 is a partial cross-sectional view of another example of a prosthesis;
FIG. 24 is a partial cross-sectional view of another example of a prosthesis;
FIG. 25 is an exploded isometric view of another example of a prosthesis in accordance with embodiments of the invention;
FIG. 26 is a top side plan view of the prosthesis illustrated in FIG. 25 when the linear actuator is moved in a distal direction to provide directional engagement with surrounding bone in accordance with some embodiments;
FIG. 27 is an exploded isometric view of another example of a prosthesis in accordance with some embodiments; and
FIG. 28 is a top side plane view of the prosthesis illustrated in FIG. 27 when the linear actuator is moved in a distal direction to provide directional engagement with the surrounding bone in accordance with some embodiments.

### DETAILED DESCRIPTION

This description of the exemplary embodiments is intended to be read in connection with the accompanying drawings, which are to be considered part of the entire written description. The drawing figures are not necessarily to scale and certain features may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. The term "operatively connected" is such an attachment, coupling or connection that allows the pertinent structures to operate as intended by virtue of that relationship.

The methods, systems, and structures described for the ankle herein may be adapted to other applications in arthroplasty, including but not limited to the knee, shoulder, hip, elbow, and other joints.

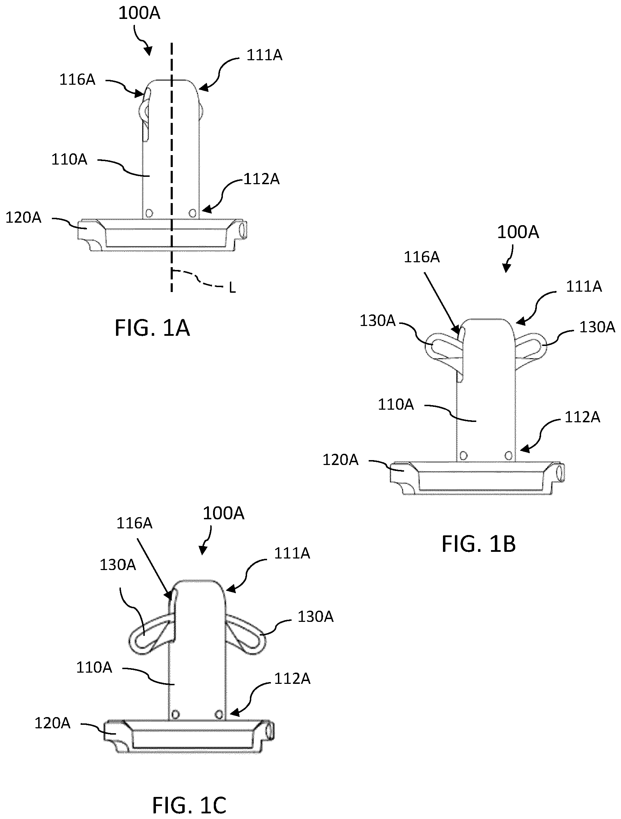

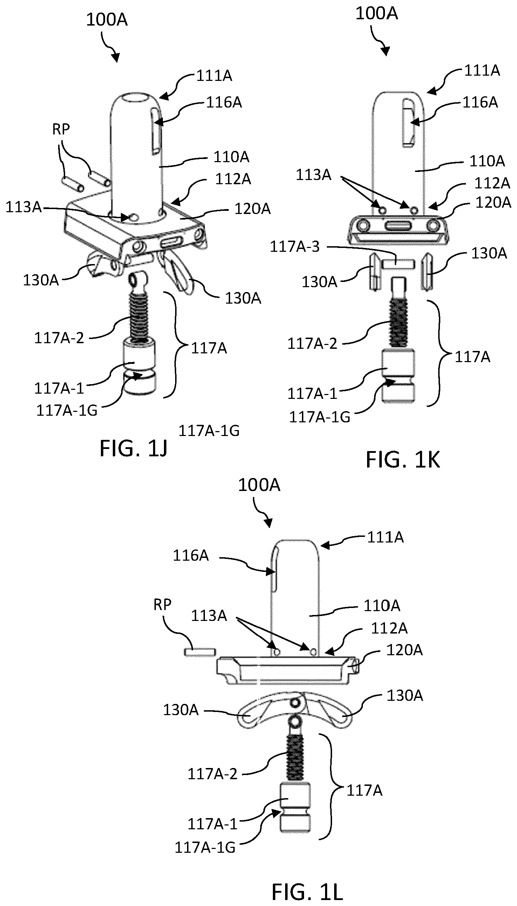

Referring to FIGS. 1A-7E, an ankle prosthesis 100A, 100B, 100C, 100D, 100E, 100F, 100G, 100H, 100I, 100J is disclosed. The ankle prosthesis comprises: a tibia stem component 110A, 110B, 110C, 110D, 110E, 110F, 110G, 110H, 110I, 110J; and a tibia tray component 120A, 120B, 120C, 120E, 120F, 120G, 120H, 120I, 120J configured to be attached to a prosthetic joint articulating surface. The tibia stem component comprises a leading end 111A, 111B, 111C, 111D, 111E, 111F, 111G, 111H, 1111, 111J a trailing end 112A, 112B, 112C, 112D, 112E, 112F, 112G, 112H, 112I, 112J and a longitudinal axis L defined therethrough.

The tibia tray component extends from the trailing end of the tibia stem component. The tibia stem component is sized and configured to be disposed in an intramedullary canal formed in a tibia. The tibia stem component comprises one or more retractable members 130A, 130B, 130C, 130D, 130E, 130F, 130G, 130H, 130I, 130J configured to be controllably movable from a retracted position and extended outward and away from the tibia stem component. In the retracted position, the one or more retractable members 130A, . . . 130J are contained substantially within the silhouette of the tibia stem component and do not extend outward. Substantially within the silhouette of the tibial stem means that a small portion (no more than about 0.5 mm) of the distal ends of the one or more retractable members can, in some embodiments, protrude out beyond the silhouette of the tibia stem. In use, after the tibia stem component is disposed in the intramedullary canal of a tibia, the one or more retractable members are moved from their retracted position outward and away from the longitudinal axis L so that the one or more retractable members engage the intramedullary canal's surrounding bone and enhance anchoring the tibia stem within the intramedullary canal.

In some preferred embodiments, the tibia stem component comprises two or more retractable members 130A, 130B, 130C, 130D, 130E, 130F, 130G, 130H, 130I, 130J. In many situations, having two or more retractable members can provide anchoring configurations that are more symmetrical. The symmetry involved here can be planar symmetry or radial symmetry with respect to the longitudinal axis L of the tibia stem component. Retraction of the retractable members 130A, 130B, 130C, 130D, 130E, 130F, 130G, 130H, 130I, 130J allows for in-situ adjustment, repositioning, and patient removal of the tibia stem component as necessary.

In some embodiments of the prosthesis 100A, 100B, 100C, 100D, 100E, 100F, 100G, 100H, 100I, 100J, the tibia tray comprises a channel 122A, 122B, 122C, 122D, 122E, 122F, 122G, 122H, 122I, 122J, extending between a pair of opposed rails 124A, 124B, 124C, 124D, 124E, 124F, 124G, 124H, 124I, 124J, for receiving the prosthetic joint surface.

In some embodiments of the prosthesis 100A, 100B, 100C, 100D, 100E, 100F, 100G, 100H, 100I, 100J, the channel in the tibia tray extends in an anterior-posterior direction, medial-lateral direction, or in an oblique direction.

In some embodiments of the prosthesis 100A, 100B, 100C, 100D, 100E, 100F, 100G, 100H, 100I, 100J, the one or more retractable members 130A, 130B, 130C, 130D, 130E, 130F, 130G, 130H, 130I, 130J extend away from the longitudinal axis L of the tibia stem component when moving from their retracted positions outward and away from the longitudinal axis L.

In some embodiments of the prosthesis 100A, 100B, 100C, 100D, 100E, 100F, 100G, 100H, 100I, 100J, the tibia stem comprises an elongated generally cylindrical shell defining an internal cavity 115A, 115B, 115C, 115D, 115E, 115F, 115G, 115H, 115I, 115J, that is open at the trailing end. An opening 116A, 116B, 116C, 116D, 116E, 116F, 116G, 116H, 116I, 116J is provided in the generally cylindrical shell of the tibia stem component for each of the one or more retractable members. The one or more retractable members extend outward through the opening in the cylindrical shell from the retracted position.

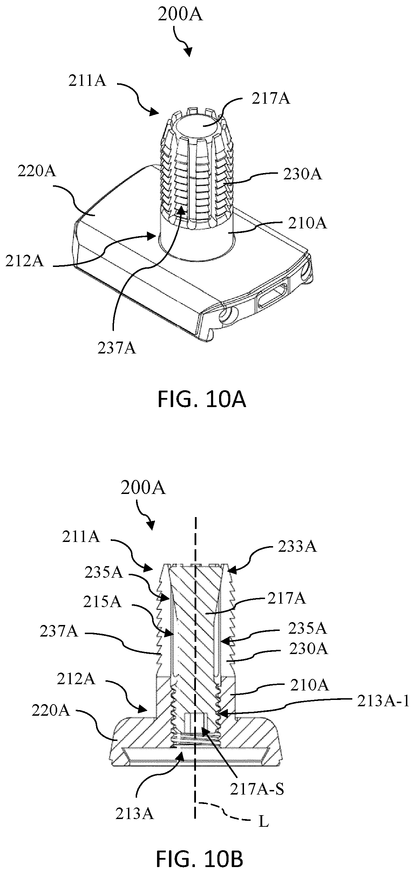

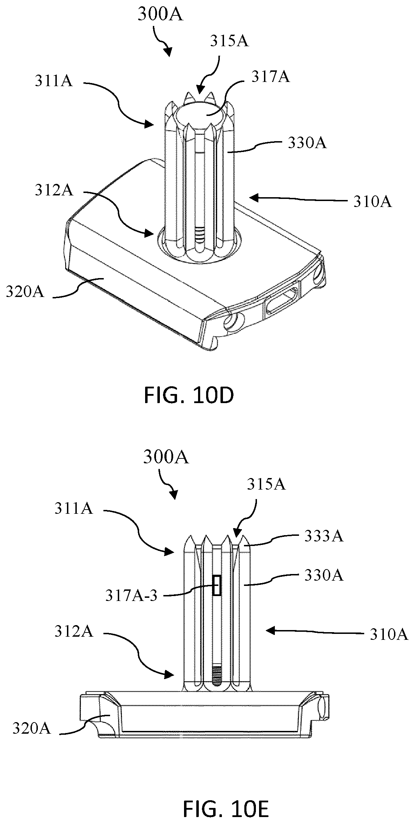

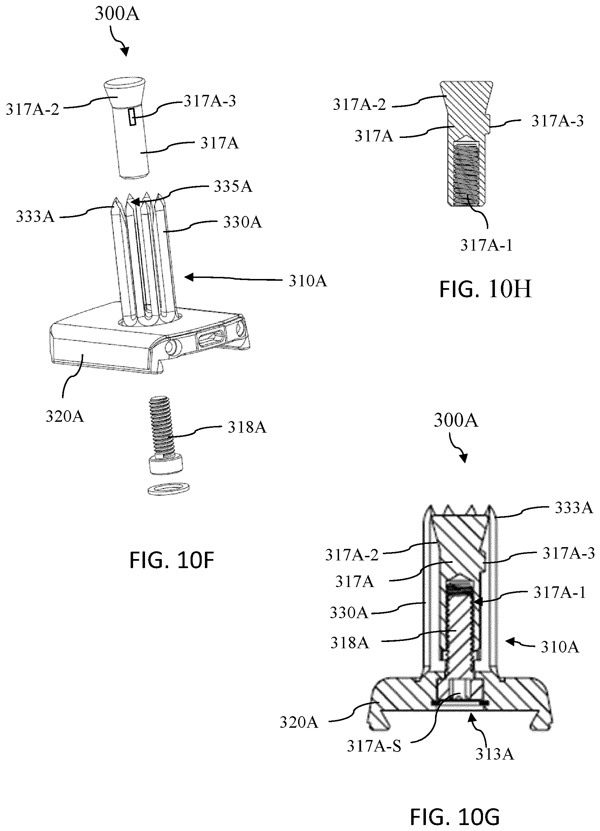

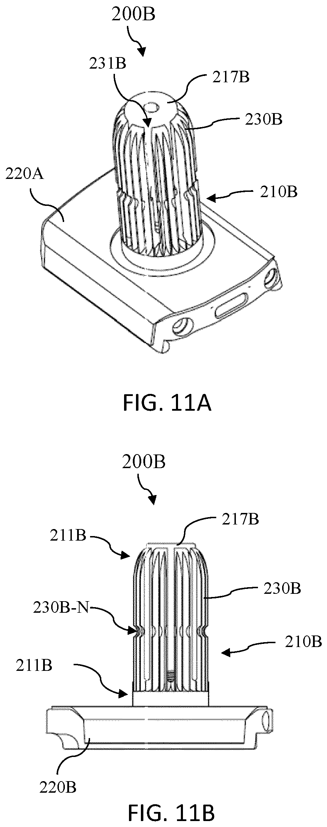

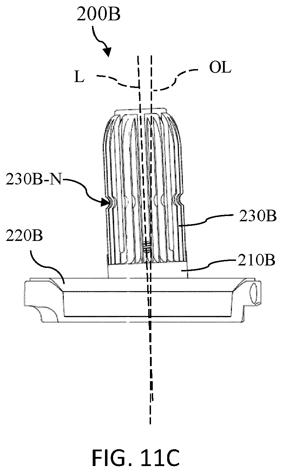

Although, the shape of the shell forming the tibia stem component is referred to as being generally cylindrical, the scope of the present disclosure encompasses a variety of shapes for the shell other than those having circular or oval cross-section. The term "generally cylindrical" as used herein is intended to encompass a structure for the shell that can have a variety of other cross-sectional shape such as polygons (i.e., a triangle, a quadrilateral, a pentagon, a hexagon, a heptagon, an octagon, etc.). Additionally, the term "generally cylindrical" as used herein is intended to encompass structures that may not have a continuous solid shell such as the shell forming the tibia stem components in the prosthesis embodiments 200A and 200B shown in FIGS. 10A and 11A, respectively. The tibial stems in prosthesis embodiments 200A and 200B do not have solid cylindrical structures but they do have generally cylindrical shapes.

In some embodiments of the prosthesis, the tibia stem further comprises a linear actuator 117A, 117B, 117C, 117D, 117E, 117F, 117G, 117H, 117I, 117J, provided within the internal cavity. The linear actuator is configured to be movable within the internal cavity along the longitudinal axis L. Each of the one or more retractable members have a distal end 131A, 131B, 131C, 131D, 131E, 131F, 131G, 131H, 131I, 131J and a proximal end 132A, 132B, 132C, 132D, 132E, 132F, 132G, 132H, 132I, 132J. The proximal end is attached to the linear actuator and the distal end is a free end that is movable through the respective opening 116A, 116B, 116C, 116D, 116E, 116F, 116G, 116H, 116I, 116J provided in the cylindrical shell of the tibia stem to engage the intramedullary canal's surrounding bone when the prosthesis is installed inside the intramedullary canal of a tibia. The movement of the one or more retractable members 130A, . . . 130J from the retracted position to being extended outward and away from the tibial stem component is controllably achieved by moving the linear actuator 117A, . . . 117J within the internal cavity 115A, ...115J. The movement of the linear actuator can be either toward the leading end 111A, . . . 111J or toward the trailing end 112A, ... 112J in the axial direction along the longitudinal axis L.

The controlled axial movement of the linear actuator can be enabled in a variety of ways. In the disclosed exemplary embodiments of the prosthesis 100A, 100B, 100C, 100D, 100E, 100F, 100G, 100H, 100I, 100J, the axial movement of the linear actuator 117A, . . . 117J is achieved by a threaded action.

For example, in the prosthesis embodiments 100A, 100B shown in FIGS. 1A - 2E, the linear actuators 117A, 117B comprise two parts: a base piece 117A-1, 117B-1 and a moving piece 117A-2, 117B-2, respectively. The base pieces 117A-1, 117B-1 are positioned in the their respective internal cavities 115A, 115B and is configured to be rotated about the longitudinal axis L but stationary so that they do not move in axial direction within the internal cavities 115A, 115B. The base pieces 117A-1, 117B-1 and the moving pieces 117A-2, 117B-2 threadedly engage each other to move the moving pieces 117A-2, 117B-2 axially (i.e., up and down) within their respective internal cavities 115A, 115B. The one or more retractable members 130A, 130B are attached to their respective moving pieces 117A-2, 117B-2 of the linear actuators 117A, 117B and by moving the moving pieces 117A-2, 117B-2 axially, the retractable members 130A, 130B can be moved from their retracted position to being extended outward away from the stem component.

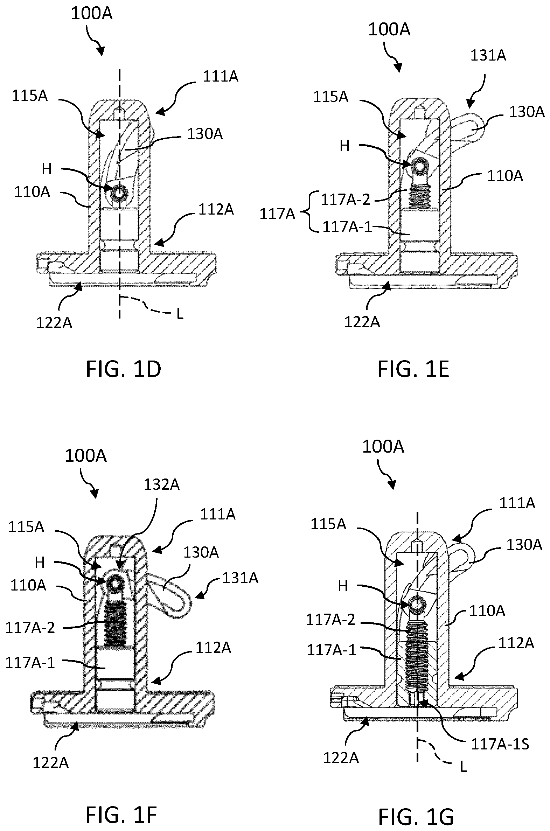

Referring to the cross-sectional view in FIG. 1G, in the prosthesis embodiment 100A, the base piece 117A-1 comprises an internally threaded hole that is open toward the leading end 111A and the threaded stem 117A-2 is received therein engaged by the mating threads. Because the base piece 117A-1 is stationary, turning the base piece 117A-1 clockwise or counter-clockwise will make the threaded stem 117A-2 to retract into away from the leading end 111A or extend out of the base piece toward the leading end 111A. This controlled up and down movement of the threaded stem 117A-2 can controllably move the one or more retractable members 130A from the retracted position shown in FIGS. 1A and 1D outward and away from the longitudinal axis L to the fully extended position shown in FIGS. 1C and 1F, and any configuration in between. FIGS. 1B, 1E, and 1G illustrate some of the intermediate positions. As previously mentioned, the one or more retractable members 130A move through the opening 116A provided in the generally cylindrical shell of the tibia stem component 110A.

Referring to FIG. 1F, in this example embodiment, the proximal end 132A of the one or more retractable members 130A are connected to the threaded stem 117A-2 by a hinged connection H. The hinged connection H can be accomplished by a pin 117A-3. The pin 117A-3 is shown in the exploded view of the prosthesis 100A in FIG. 1K. This hinged connection enables the threaded stem 117A-2 to move the one or more retractable members 130A from their retracted position and extend them through the respective openings 116A. Each of the one or more retractable members 130A has a curved shape that facilitates the extending outward movement as the threaded stem 117A-2 pushes the proximal end 132A of the retractable member 130A upward. Referring to FIGS. 1A and 1D, as the proximal end 132A of the retractable member 130A is pushed up by the threaded stem 117A-2, the top edge of the retractable member 130A is urged against the top edge of the opening 116A which guides the retractable member 130A to extend outward as the retractable member 130A swivels about the hinged joint H.

The screw threads on the base piece 117A-1 and the threaded stem 117A-2 can be matched to be either left-handed or right-handed which will determine which direction the base piece 117A-1 needs to be turned to raise or lower the threaded stem 117A-2.

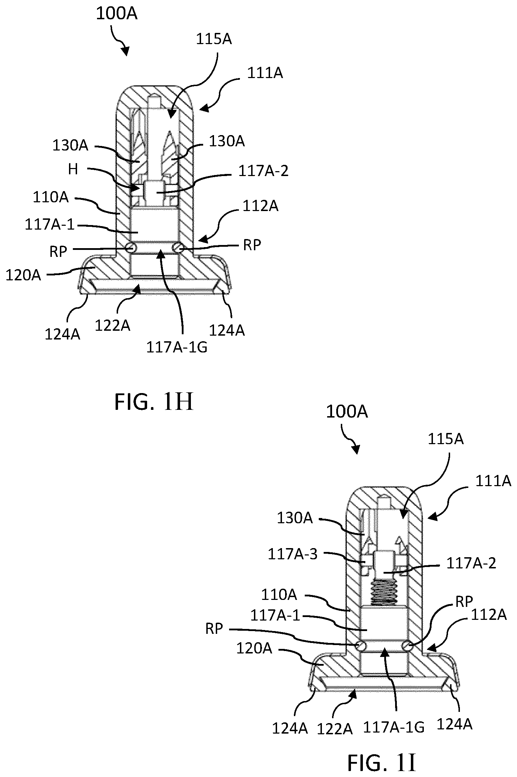

In the illustrated example, the base piece 117A-1 is configured to stay stationary in the axial direction while still being rotatable within the internal cavity 115A by a pair of retaining pins RP. This can be better seen in FIGS. 1H-1L. The base piece 117A-1 is configured with an annular groove 117A-1G and the substantially cylindrical shell of the tibia stem component 110A comprises two generally parallel through holes 111A for receiving the retaining pins RP near the trailing end 112A. The two through holes 111A are positioned such that the retaining pins RP extend through the two through holes 111A and are aligned with the annular groove 117A-1G of the base piece 117A-1 so that the retaining pins RP intercept the groove from two opposing sides as shown in the cross-sectional views in FIGS. 1H and 1I, and hold the base piece 117A-1 in place within the internal cavity 115A. In some embodiments, the base piece 117A-1 is stationary near the trailing end 112A within the internal cavity and the moving piece 117A-2 is movable along the longitudinal axis L of the tibia stem component with respect to the base piece by operation of the threaded engagement.

The base piece 117A-1 can be provided with a tool-receiving socket 117A-1S (See FIG. 1G) at the bottom end so that a tool such as a wrench or a screwdriver can be used to turn the base piece 117A-1 and control the movement of the one or more retractable members 130A.

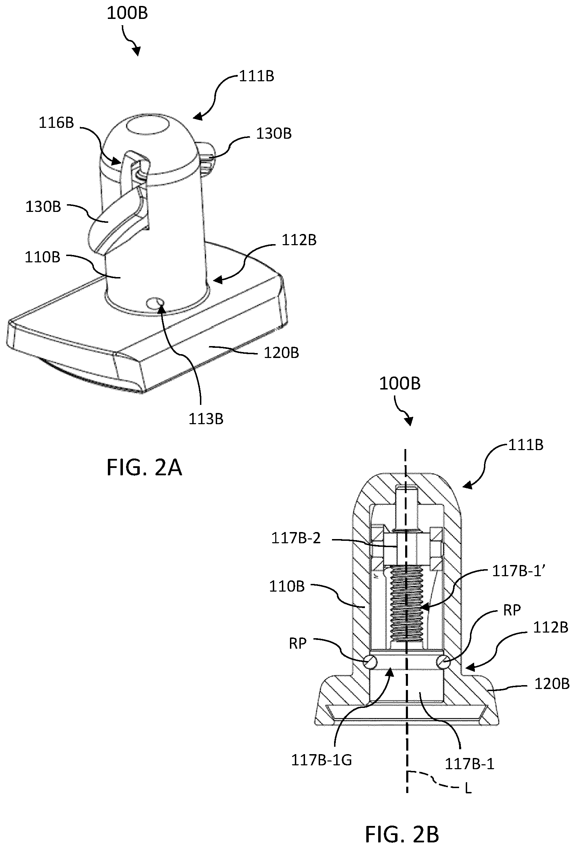

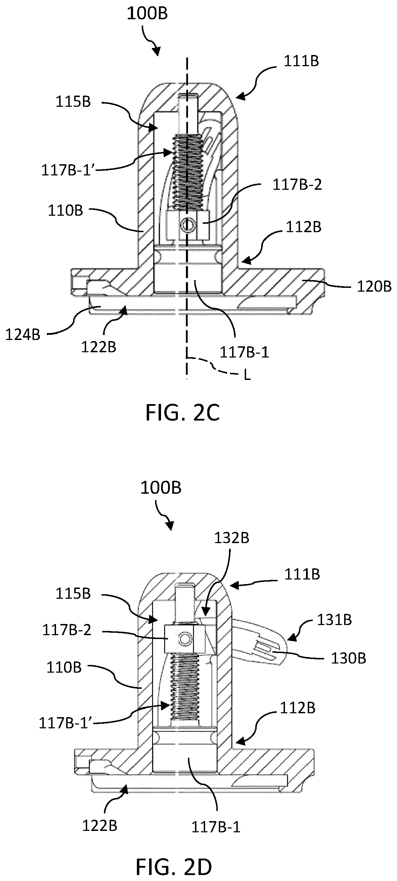

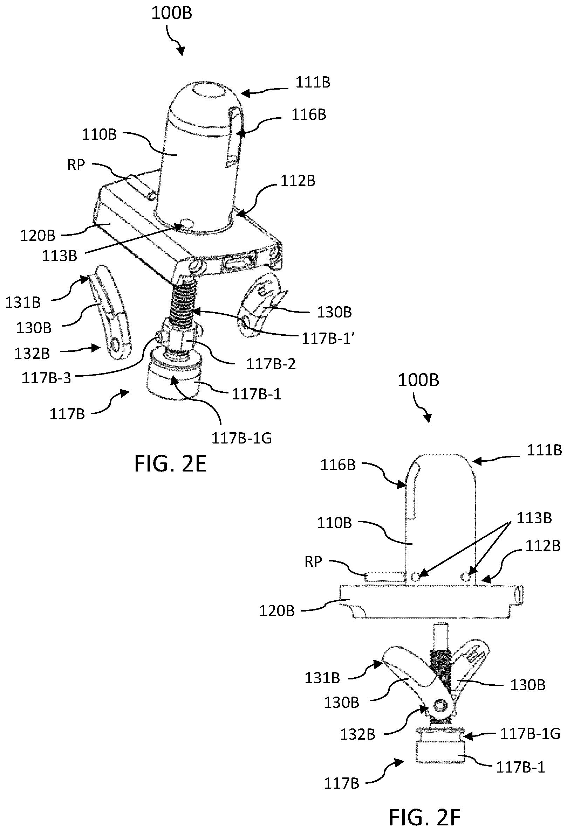

Referring to the cross-sectional views in FIGS. 2B-2D, in the prosthesis embodiment 100B, the base piece 117B-1 comprises a threaded stem portion 117B-1' and the moving piece 117B-2 is a threaded nut structure that threadedly engages the threaded stem portion 117B-1'. In other words, the threaded stem portion 117B-1' is a leadscrew and the moving piece 117B-2 is the leadscrew nut that translates the rotational motion of the leadscrew into a linear motion. The proximal ends 132B of the one or more retractable members 130B are connected to the moving piece 117B-2. Because the base piece 117B-1 is stationary, by turning the base piece 117B-1 clockwise or counter-clockwise, the moving piece 117B-2 can be moved up or down the threaded stem portion 117B-1' by operation of their threaded engagement. This controlled up and down movement of the moving piece 117B-2 can controllably move the one or more retractable members 130B from their retracted position shown in FIG. 2C outward and away from the longitudinal axis L to their fully extended position shown in FIG. 2D, and any configuration in between. As previously mentioned, the one or more retractable members 130B move through the opening 116B provided in the generally cylindrical shell of the tibia stem component 110B.

Referring to FIG. 2D, in this example embodiment, the proximal end 132B of the one or more retractable members 130B are connected to the moving piece 117B-2 by a hinged connection H. The hinged connection H can be accomplished by connecting pins 117B-3 provided on the moving piece 117B-2. The connecting pins 117B-3 are shown in the exploded view of the prosthesis 100B in FIG. 2E. This hinged connection enables the moving piece 117B-2 to move the one or more retractable members 130B from their retracted position and extend them through the respective openings 116B. Each of the one or more retractable members 130B has a curved shape that facilitates the extending outward movement as the moving piece 117B-2 pushes the proximal end 132B of the retractable member 130B upward. Referring to FIGS. 2C and 2D, as the proximal end 132B of the retractable member 130B is pushed up by the moving piece 117A-2, the top edge of the retractable member 130B is urged against the top edge of the opening 116B which guides the retractable member 130B to extend outward as the retractable member 130B swivels about the hinged joint H.

The screw threads on the threaded stem portion 117B-1' and the moving piece 117B-2 can be matched to be either left-handed or right-handed which will determine which direction the base piece 117B-1 needs to be turned to raise or lower the moving piece 117B-2.

The base piece 117B-1 can be provided with a tool-receiving socket (not shown) similar to the tool-receiving socket 117A-1S of the base piece 117A-1 at the bottom end so that a tool such as a wrench or a screwdriver can be used to turn the base piece 117B-1 and control the movement of the one or more retractable members 130B.

In the illustrated example, the base piece 117B-1 is configured to stay stationary in the axial direction while still being rotatable within the internal cavity 115B by a pair of retaining pins RP. The retaining pins RP works the same way as in the prosthesis 100A. This can be better seen in the cross-sectional view in FIG. 2B and the exploded views FIGS. 2E-2F. The base piece 117B-1 is configured with an annular groove 117B-1G and the substantially cylindrical shell of the tibia stem component 110B comprises two generally parallel through holes 111B for receiving the retaining pins RP near the trailing end 112B. The two through holes 111B are positioned such that the retaining pins RP extend through the two through holes 111B and are aligned with the annular groove 117B-1G of the base piece 117B-1 so that the pins RP intercept the groove from two opposing sides as shown in the cross-sectional views in FIGS. 1H and 1I, and hold the base piece 117B-1 in place within the internal cavity 115B.

In some embodiments of the prosthesis 100C, 100D, 100E, 100F, 100G, 100H, the proximal ends 132C, . . . 132H of the one or more retractable members 130C, . . . 130H are attached to the substantially cylindrical shell of the tibia stem 110C, . . . 110H and the distal end being a free end that engages the intramedullary canal's surrounding bone. In preferred embodiments, the one or more retractable members 130C, . . . 130H are hingeably attached to the substantially cylindrical shell of the tibia stem.

In some embodiments of the prosthesis 100C, 100D, 100E, 100F, 100G, 100H, the tibia stem 110C, ...110H further comprises a linear actuator 117C, . . . 117H provided within the internal cavity 115C, . . . 115H. The linear actuator 117C, . . . 117H is configured to be axially movable within the internal cavity along the longitudinal axis L. Each of the one or more retractable members 130C, ...130H have a distal end 131C, ... 131H and a proximal end 132C, . . . 132H, and the proximal end is attached to the substantially cylindrical shell of the tibia stem 110C, . . . 110H. The distal end 131C, ...131H a free end that engages the intramedullary canal's surrounding bone. In preferred embodiments, the proximal end 132C, . .. 132H is hingeably attached to the substantially cylindrical shell of the tibial stem.

In some embodiments of the prosthesis 100C, 100D, 100E, 100F, 100G, 100H, the movement of the linear actuator 117C, . . . 117H toward the leading end 111C, . . . 111H of the tibia stem 110C, . . . 110H enables the one or more retractable members to move outward and away from the longitudinal axis L. In some embodiments, the linear actuator and the internal cavity's sidewall engage each other for moving the linear actuator within the internal cavity 115C, ... 115H. In some embodiments, the linear actuator 117C, . . . 117H and the internal cavity's sidewall threadedly engage each other.

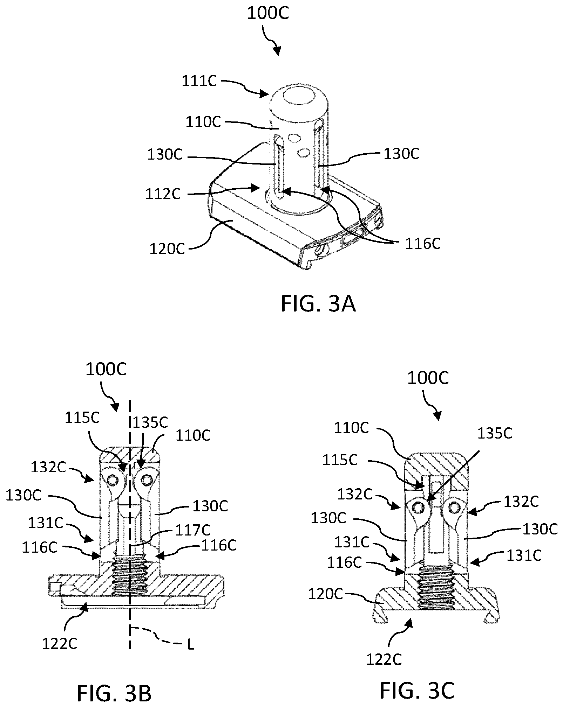

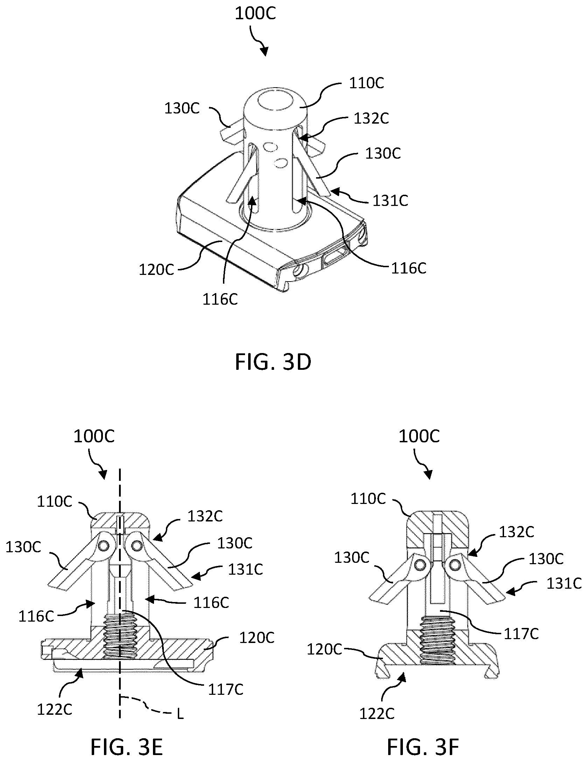

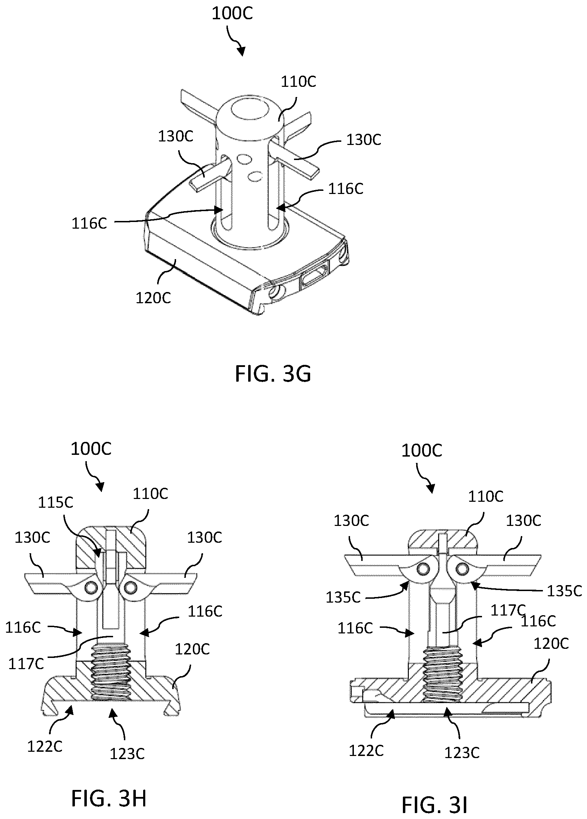

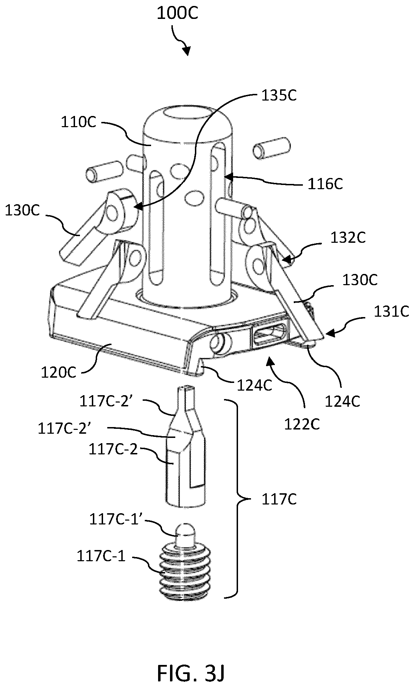

For example, in the prosthesis embodiment 100C shown in FIGS. 3A-3J, the proximal end 132C of each of the one or more retractable members 130C comprises a cam surface 135C (see FIGS. 3I and 3J) that is configured to engage the linear actuator 117C. The linear actuator 117C comprises a base piece 117C-1 and a top piece 117C-2. As shown in the cross-sectional views shown in FIGS. 3E, 3F, 3H, and 3I, the top piece 117C-2 and the base piece 117C-1 are situated within the internal cavity 115C with the top piece 117C-2 above the base piece 117C-1. As shown in the exploded view in FIG. 3J, the top piece and the base piece are assembled in such a way so that the base piece 117C-1 can be rotated about the longitudinal axis L relative to the top piece 117C-2. In the illustrated example, the base piece 117C-1 comprises an axially located pin 117C-1' that fits into a mating hole (not shown) provided on the top piece 117C-2. In other embodiments, the location of the pin 117C-1' can be reversed so that the top piece 117C-2 comprises the pin and the base piece 117C-1 comprises the mating hole.

The base piece 117C-1 is threaded on its outer surface and the sidewall of the internal cavity 115C is threaded so that the base piece 117C-1 and the tibia stem component 110C threadedly engage each other. By turning the base piece 117C-1 clockwise or counterclockwise, the base 117C-1 can be moved up or down within the internal cavity 115C by operation of their threaded engagement. When the base piece 117C-1 moves up into the internal cavity 115C, it pushes the top piece 117C-2 upward. As the top piece 117C-2 moves up, the leading end of the top piece 117C-2 is a cam engaging surface and contacts the cam surface 135C of the one or more retractable members 130C and moves the one or more retractable members 130C from their retracted position shown in FIG. 3C outward and away from the longitudinal axis L to their fully extended position shown in FIG. 3H, and any configuration in between. As previously mentioned, the one or more retractable members 130C move through the opening 116C provided in the generally cylindrical shell of the tibia stem component 110C.

As used herein, the terms "cam surface" and "cam engaging surface" refer to any paired surfaces on two components, one on each component, that come in contact with each other and operate to result in urging one or both of the two components to move in an intended manner. Thus, one of the two paired surfaces is referred to as the cam surface and the corresponding surface on the other component is referred to as the cam engaging surface.

The prosthesis 100C comprises two pairs of retractable members 130C. The retractable members 130C in each pair are positioned opposite from each other, as shown in FIGS. 3A, 3D, and 3G, and the leading end of the top piece 117C-2 extends between two retractable members 130C in each pair. As shown in the cross-sectional views FIGS. 3B, 3C, 3E, 3F, 3H, and 3I, the leading end of the top piece 117C-2 is in contact with the cam surface 135C of the retractable members 130C. As shown in FIG 3J, the leading end of the top piece 117C-2 comprises two pairs of slanted surfaces 117C-2' and 117C-2" and each pair contacts the corresponding pairs of the cam surfaces 135C of the retractable members 130C. The movement of the retractable members 130C from their retracted position outward and away from the longitudinal axis L is enabled by the engagement between the slanted surfaces 117C-2' and 117C-2" and the cam surfaces 135C. As the linear actuator assembly 117C is raised toward the leading end 111C of the tibia stem component, the two pairs of the slanted surfaces 117C-2' and 117C-2" wedged between the two pairs of the cam surfaces contact the cam surfaces 135C and pushes each of the retractable members to move from the retracted position outward and away from the longitudinal axis L.

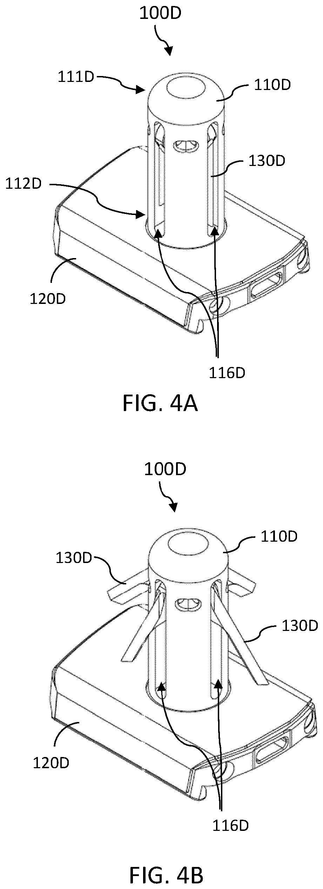

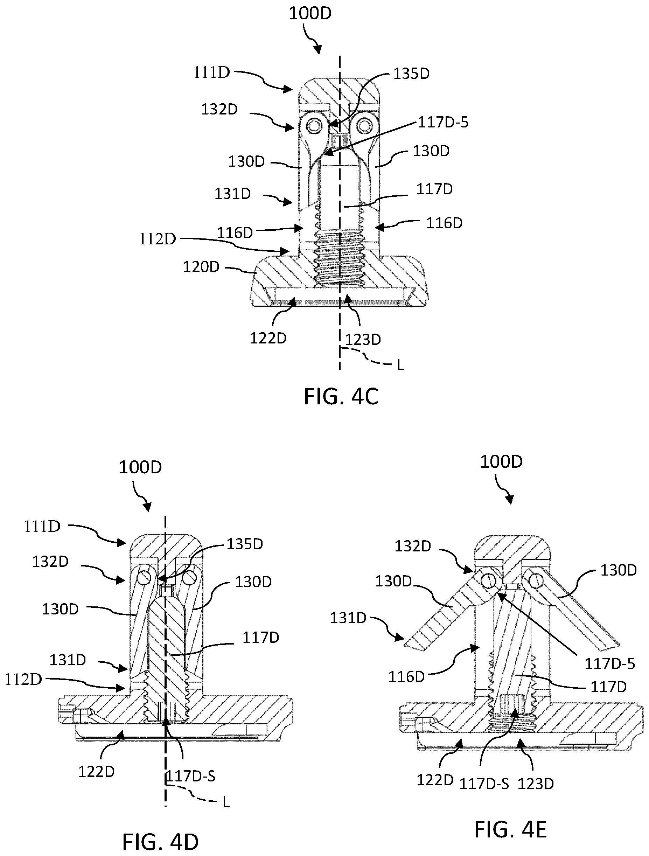

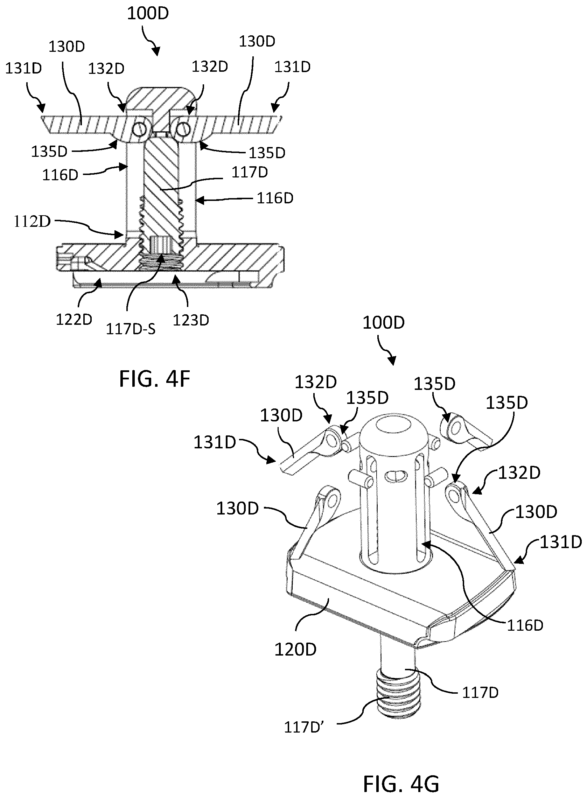

Referring to FIGS. 4A-4G, in the prosthesis embodiment 100D, the linear actuator 117D comprises externally threaded surface 117D' (See FIG. 4G) that engages the corresponding threads on the sidewall of the internal cavity 115D. By turning the linear actuator 117D clockwise or counterclockwise, the linear actuator 117D can be moved up or down within the internal cavity 115D by operation of their threaded engagement. As the linear actuator 117D moves up, the leading end of the linear actuator 117D contacts the cam surface 135D of the one or more retractable members 130D and moves the one or more retractable members 130D from their retracted position shown in FIG. 4C outward and away from the longitudinal axis L toward the fully extended position shown in FIG. 4F, and any configuration in between. The linear actuator 117D can be provided with a tool-receiving socket 117D-S (See FIGS. 4D, 4E, 4F) on the bottom end (the end near the trailing end 112D) that is accessible through the opening 123D at the trailing end 112D so that a tool such as a wrench, a screwdriver, etc. can be used to turn the linear actuator 117D. The one or more retractable members 130D move through the opening 116D provided in the generally cylindrical shell of the tibia stem component 110D.

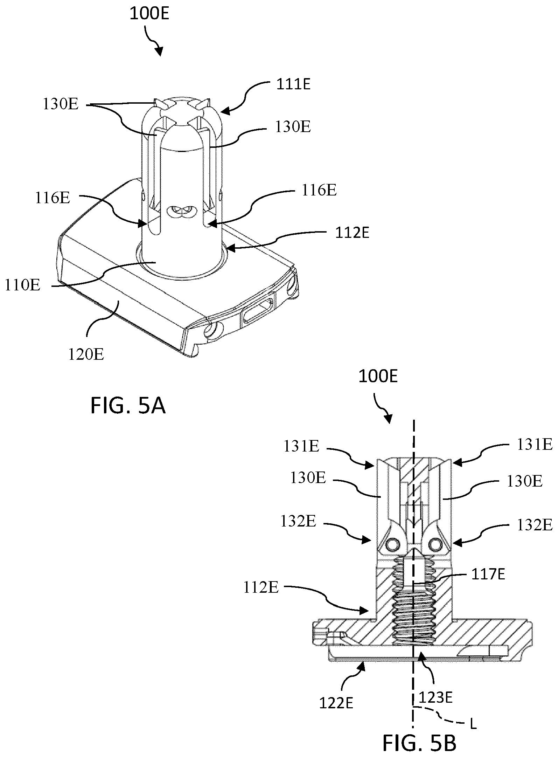

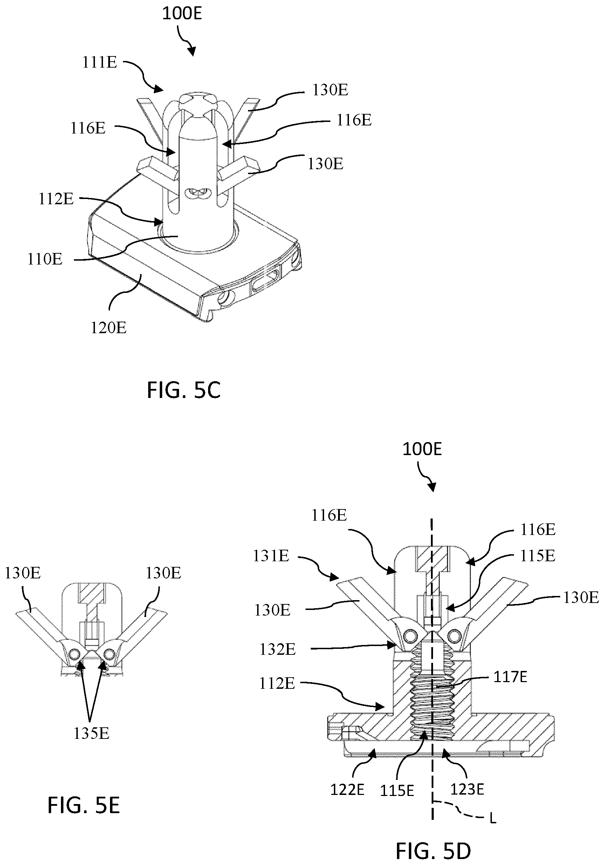

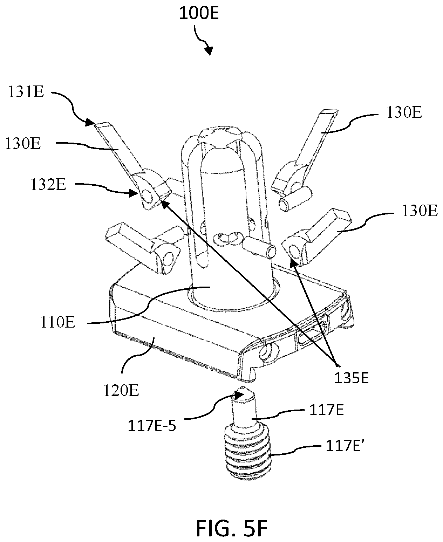

Referring to FIGS. 5A-5F, in the prosthesis embodiment 100E, the linear actuator 117E comprises externally threaded surface 117E' (See FIG. 5E) that engages the corresponding threads on the sidewall of the internal cavity 115E. The linear actuator 117E also can be provided with a tool-receiving socket similar to the socket 117D-S on the bottom end that is accessible through the opening 123E at the trailing end 112E so that a tool such as a wrench, a screwdriver, etc. can be used to turn the linear actuator 117E.

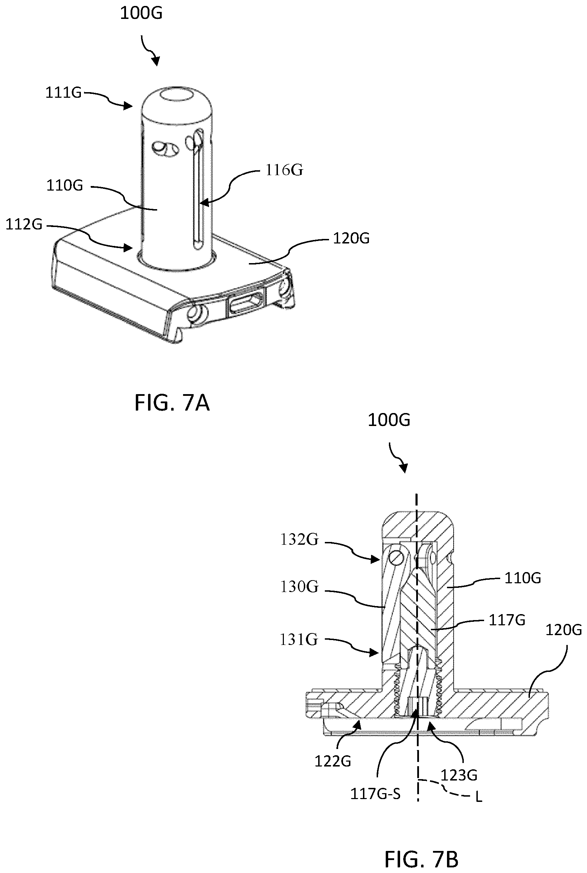

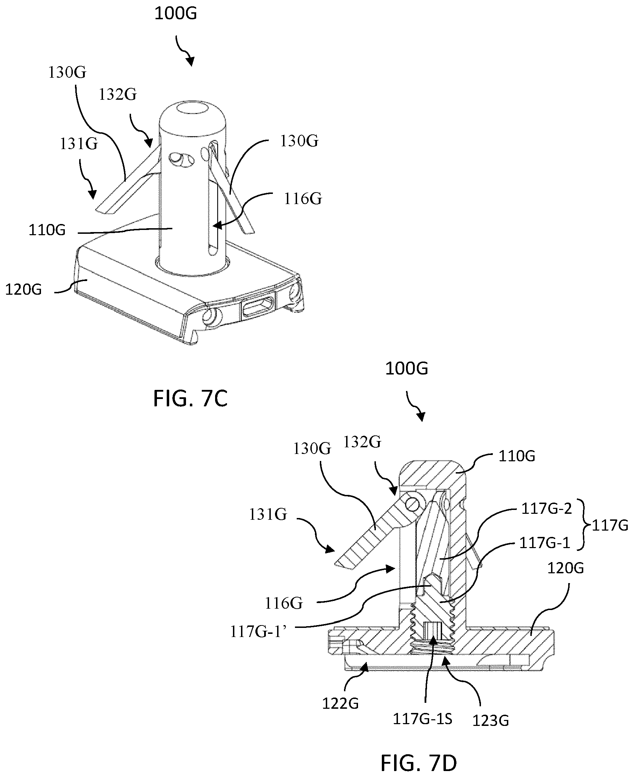

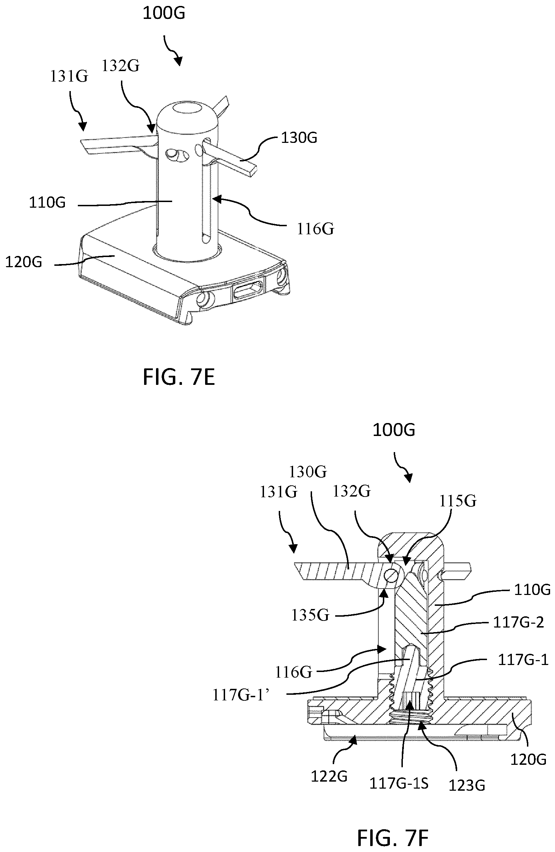

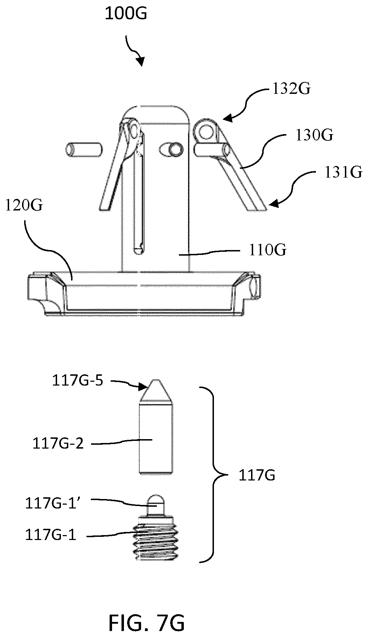

Referring to FIGS. 7A-7G, in the prosthesis embodiment 100G, the linear actuator 117G comprises a base piece 117G-1 and a top piece 117G-2. This structure is similar to the two-piece configuration of the linear actuator 117G described above. The base piece 117G-1 is threaded on its outer surface and the sidewall of the internal cavity 115G is threaded so that the base piece 117G-1 and the tibia stem component 110G threadedly engage each other. By turning the base piece 117G-1 clockwise or counterclockwise, the base 117G-1 can be moved up or down within the internal cavity 115G by operation of their threaded engagement. When the base piece 117G-1 moves up into the internal cavity 115G, it pushes the top piece 117G-2 upward. As the top piece 117G-2 moves up, the leading end of the top piece 117G-2 contacts the camming surface 135G of the one or more retractable members 130G and moves the one or more retractable members 130G from their retracted position shown in FIG. 7B outward and away from the longitudinal axis L to their fully extended position shown in FIG. 7F, and any configuration in between. As previously mentioned, the one or more retractable members 130G move through the opening 116G provided in the generally cylindrical shell of the tibia stem component 110G.

In some embodiments of the prosthesis 100C, 100D, 100E, 100G, 100H the linear actuator 117C, 117D, 117E, 117G, 117H comprises a leading end and a trailing end corresponding with the direction of the tibia stem's leading end 111C, 111D, 111E, 111G, 111H and trailing end 112C, 112D, 112E, 112G, 112H, and the linear actuator further comprises a cam engaging surface 117C-2' 117D-5, 117E-5, 117G-5, 117H-5 near its leading end, and each of the proximal ends of the two or more retractable members comprises a cam surface 135C, 135D, 135E, 135G, 135H that engages the cam engaging surface that enables the one or more retractable members to move outward and away from the longitudinal axis L when the linear actuator moves toward the leading end of the tibia stem.

In some embodiments of the prosthesis, the distal surface 131A, . . . 131H of each of the one or more retractable members 130A, ... 130H comprise a plurality of surface features that are configured to engage the intramedullary canal's surrounding bone and enhance anchoring the tibia stem within the intramedullary canal. Such surface features can be any surface feature such as surface texturing or teeth having a biased orientation.

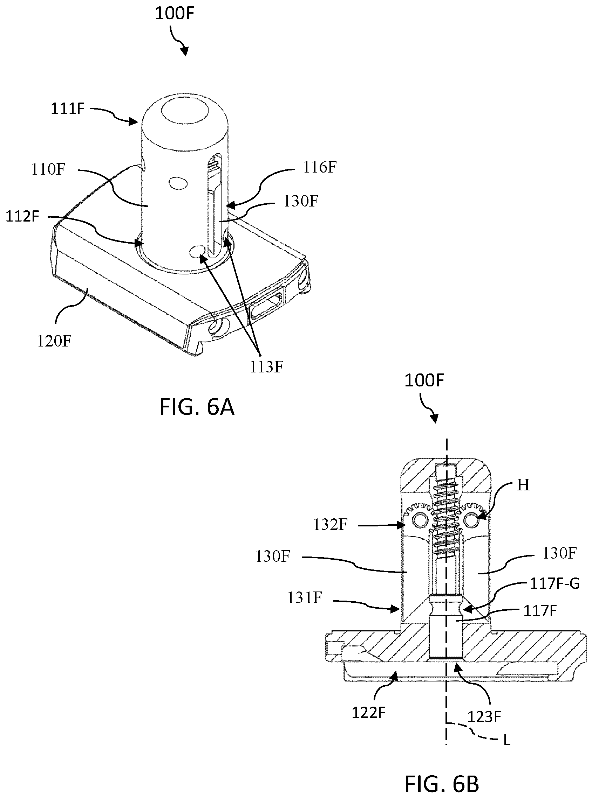

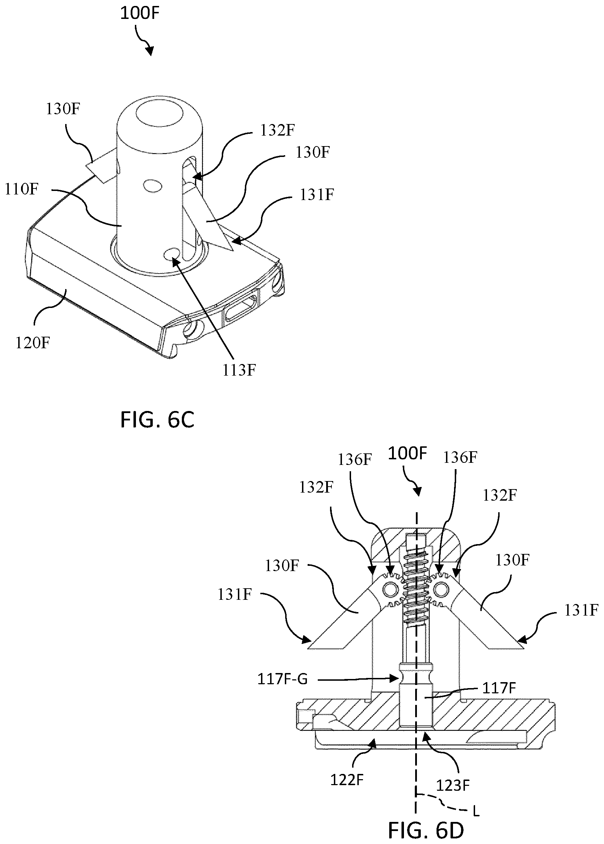

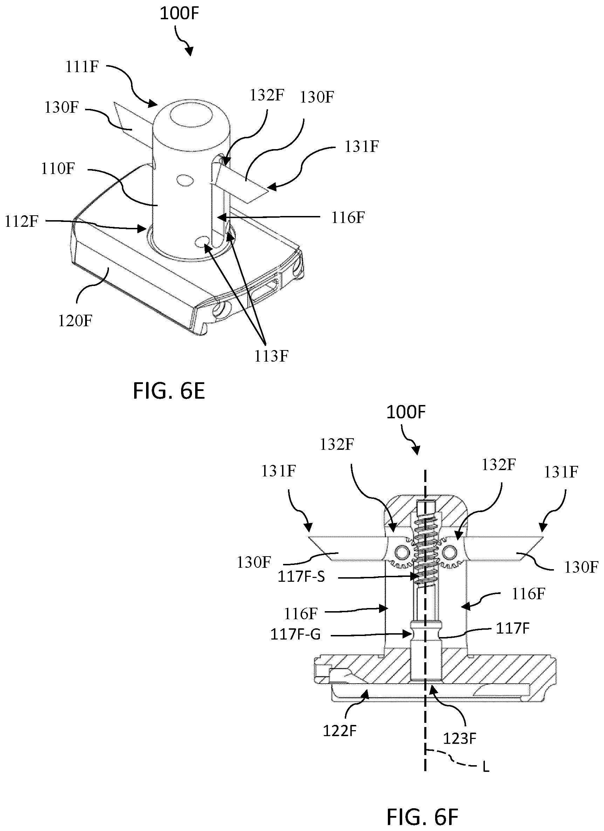

Referring to FIGS. 6A-6G, according to some embodiments, disclosed is a prosthesis 100F, wherein the tibia stem component 110F further comprises an actuator 117F provided within the internal cavity 115F. The actuator 117F is configured to be rotatable about the longitudinal axis L within the internal cavity. Each of the one or more retractable members 130F have a distal end 131F and a proximal end 132F, the proximal end being attached to the tibia stem component 110F and the distal end being a free end that engages the intramedullary canal's surrounding bone. The one or more retractable members 130F are moved from the retracted position and extended outward and away from the longitudinal axis L by rotating the actuator 117F within the internal cavity.

In some embodiments of the prosthesis 100F, the actuator 117F comprises a leadscrew portion 117F-S and the proximal end of each of the one or more retractable members comprises a toothed gear portion 136F that is engaged with the leadscrew portion. Rotating the actuator 117F engages the leadscrew 117F-S and the toothed gear portion 136F for moving the one or more retractable members from the retracted position.

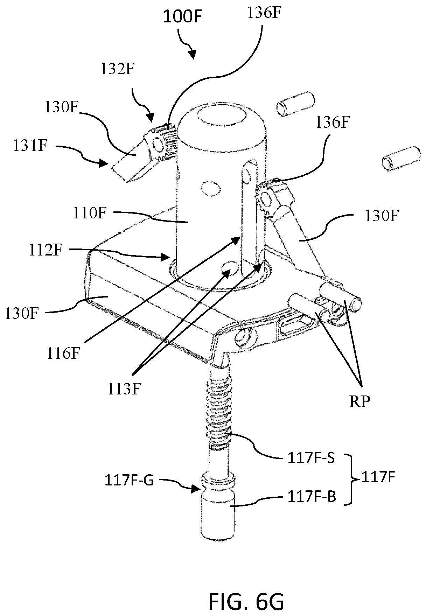

As shown in the cross-sectional views in FIGS. 6B, 6D, and 6F, the actuator 117F is positioned within the internal cavity 115F aligned with the longitudinal axis L. The actuator 117F comprises the leadscrew portion 117F-S and a base portion 117F-B. The base portion 117F-B is provided with an annular groove 117F-G for holding the actuator 117F in the internal cavity 115F while allowing it to rotate. This is achieved by a configuration similar to the way the base piece 117A-1 of the prosthesis embodiment 100A is held within its internal cavity 115A. The actuator 117F is held in place by two retaining pins RP (see FIG. 6G) inserted through two substantially parallel through holes 113F provided in the tibia stem component 110F near the trailing end 112F. The retaining pins RP engage the annular groove 117F-G from substantially the opposite sides and hold the actuator 117F in place while allowing the actuator 117F to rotate about the longitudinal axis L.

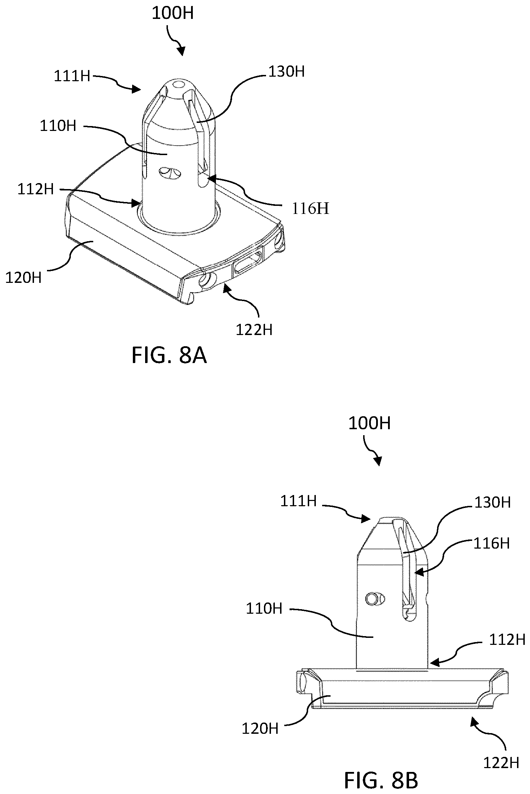

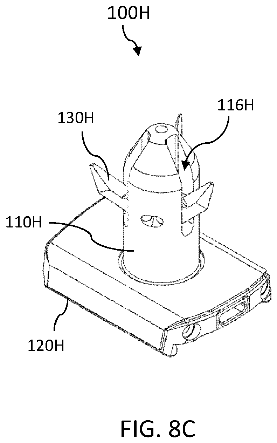

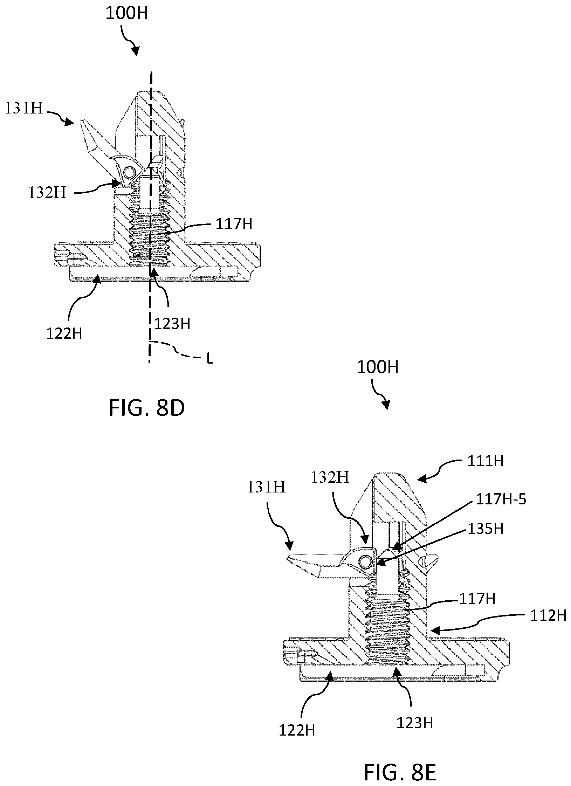

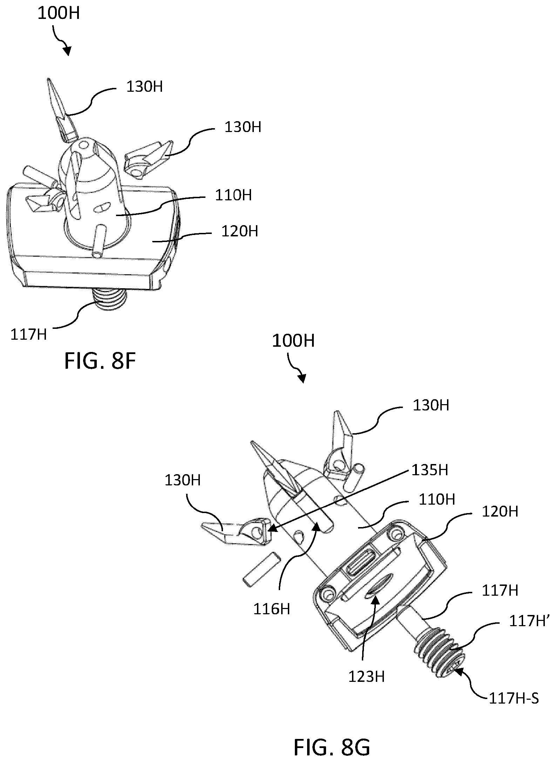

Referring to FIGS. 8A-8G, in the prosthesis embodiment 100H, the linear actuator 117H comprises externally threaded surface 117H' (See FIG. 8G) that engages the corresponding threads on the sidewall of the internal cavity 115H. By turning the linear actuator 117H clockwise or counterclockwise, the linear actuator 117H can be translated up or down within the internal cavity 115H by operation of their threaded engagement. As the linear actuator 117H moves up, the leading end of the linear actuator 117H contacts the cam surface 135H of the one or more retractable members 130H and moves the one or more retractable members 130H from their retracted position shown in FIG. 8B outward and away from the longitudinal axis L toward the fully extended position shown in FIG. 8E, and any configuration in between. The linear actuator 117H also can be provided with a tool-receiving socket 117H-S (See FIG. 8G) on the bottom end (the end near the trailing end 112H) that is accessible through the opening 123H (See FIG. 8G) at the trailing end 112H so that a tool such as a wrench, a screwdriver, etc. can be used to turn the linear actuator 117H. The one or more retractable members 130H move through the opening 116H provided in the generally cylindrical shell of the tibia stem component 110H.

In addition to the improved securement of the tibia stem component in the intramedullary canal, one additional beneficial feature of the prosthesis embodiments 100A, 100B, 100E, 100H is that the actuation direction of the one or more retractable members 130A, 130B, 130E, and 130H as they extend outward urges the tibia stem component further into the intramedullary canal. In this case, the tibial tray is drawn into the distal tibial cut surface at the ankle joint.

In the embodiments of the prosthesis 100E, 100H, the distal ends 131E, 131H of the one or more retractable members 130E, 130H extend toward the leading end 111E, 111H of the tibia stem 110E, 110H when in the retracted position.

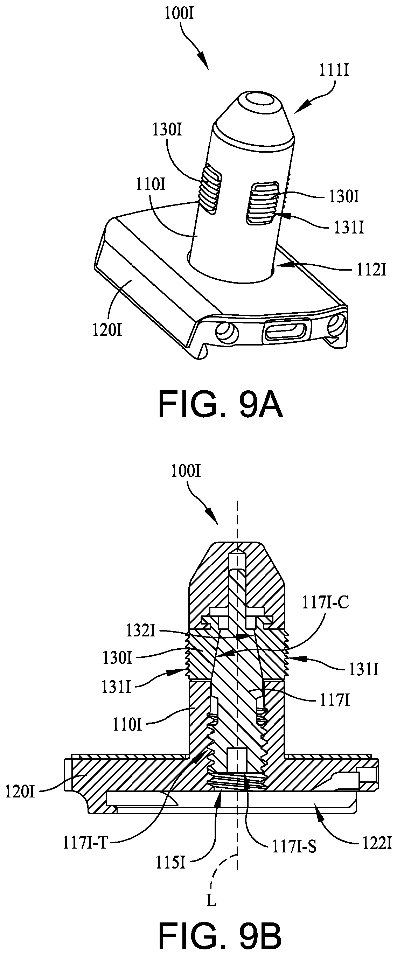

Referring to FIGS. 9A-9C, in the prosthesis embodiment 100I, the tibia stem component 110I comprises a linear actuator 117I provided within the internal cavity 115I. The linear actuator 117I is configured to be movable within the internal cavity 115I along the longitudinal axis L. The one or more retractable members 130I are moved from the retracted position shown in FIG. 9B and extended outward and away from the longitudinal axis L by translating the linear actuator 117I within the internal cavity 115I.

In some embodiments, the linear actuator 117I and the internal cavity's sidewall threadedly engage each other for translating the linear actuator 117I within the internal cavity. A portion of the linear actuator 117I is provided with male threads 117I-T and the sidewall of the internal cavity 115I is provided with corresponding female threads to achieve the threaded engagement. The bottom end of the linear actuator 117I can be configured with a tool-receiving socket 117I-S so that a tool such as a wrench or a screwdriver can be used to turn the linear actuator 117I and control the movement of the one or more retractable members 130I.

In some embodiments of the prosthesis 100I, translation of the linear actuator 117I toward the leading end 111I of the tibia stem component 110I enables the one or more retractable members 130I to extend outward.

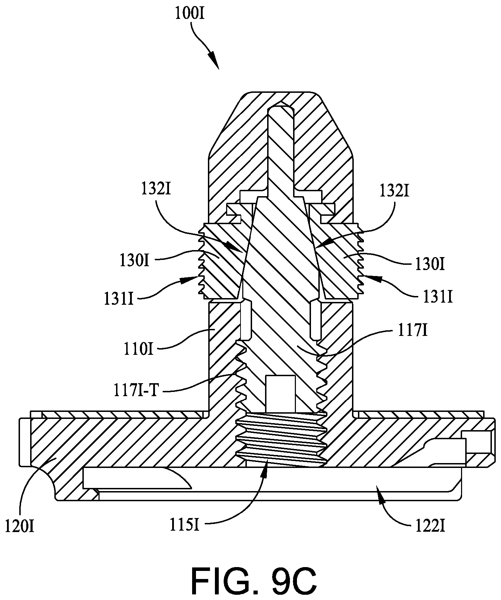

In some embodiments of the prosthesis 100I, the linear actuator 117I comprises a leading end and a trailing end corresponding with the direction of the tibia stem's leading end 111I and trailing end 112I, and further comprises a cam engaging surface 117I-C near its leading end. Each of the one or more retractable members 130I comprises a distal surface 131I and a proximal surface 132I with respect to the longitudinal axis L of the tibia stem component, wherein the proximal surface 132I of each of the one or more retractable members 130I is a cam surface that engages the cam engaging surface 117I-C of the linear actuator. When the linear actuator 117I is translated upward toward the leading end 111I, the cam engaging surface 117I-C pushes against the cam surface 132I (i.e. the proximal surface) and enables the one or more retractable members 130I to be pushed from their retracted position shown in FIGS. 9A-9B outward and away from the longitudinal axis L toward the fully extended position shown in FIG. 9C, and any configuration in between.

In some embodiments of the prosthesis 100I, the distal surface 1311 of each of the one or more retractable members 130I comprises a plurality of surface features that are configured to engage the intramedullary canal's surrounding bone and enhance anchoring the tibia stem component 110I within the intramedullary canal. In the illustrated example shown in FIGS. 9A-9B, the plurality of surface features on the distal surface 131I are teeth having a biased orientation.

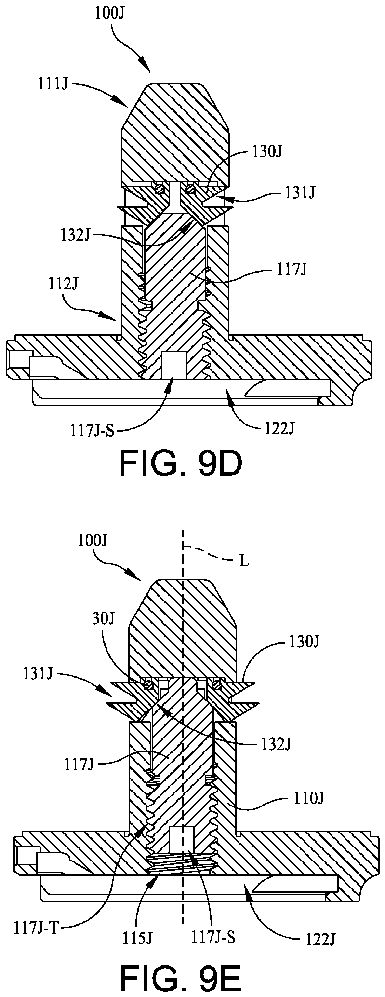

Referring to FIGS. 9D-9F, in the prosthesis embodiment 100J, the tibia stem component 110J comprises a linear actuator 117J provided within the internal cavity 115J. The linear actuator 117J is configured to be movable within the internal cavity 115J along the longitudinal axis L. Two or more retractable members 130I provided in the tibia stem component 110J are moved from the retracted position shown in FIG. 9B and extended outward and away from the longitudinal axis L by translating the linear actuator 117J within the internal cavity 115J.

In some embodiments, the linear actuator 117J and the internal cavity's sidewall threadedly engage each other for translating the linear actuator 117J within the internal cavity. A portion of the linear actuator 117J is provided with male threads 117J-T and the sidewall of the internal cavity 115J is provided with corresponding female threads to achieve the threaded engagement. The bottom end of the linear actuator 117J can be configured with a tool-receiving socket 117J-S so that a tool such as a wrench or a screwdriver can be used to turn the linear actuator 117J and control the movement of the two or more retractable members 1301.

In some embodiments of the prosthesis 100J, translation of the linear actuator 117J toward the leading end 111J of the tibia stem component 110J enables the two or more retractable members 130J to extend outward.

In some embodiments of the prosthesis 100J, the linear actuator 117J comprises a leading end and a trailing end corresponding with the direction of the tibia stem's leading end 111J and trailing end 112J, and further comprises a cam engaging surface 117J-C near its leading end. Each of the two or more retractable members 130J comprises a distal surface 131J and a proximal surface 132J with respect to the longitudinal axis L of the tibia stem component, wherein the proximal surface 132J of each of the two or more retractable members 130J is a cam surface that engages the cam engaging surface 117J-C of the linear actuator. When the linear actuator 117J is translated upward toward the leading end 111J, the cam engaging surface 117J-C pushes against the cam surface 132J (i.e. the proximal surface) and enables the two or more retractable members 130J to be pushed from their retracted position shown in FIG. 9D outward and away from the longitudinal axis L toward the fully extended position shown in FIG. 9E, and any configuration in between.

In some embodiments of the prosthesis 100J, the distal surface 131J of each of the two or more retractable members 130J comprises a plurality of surface features that are configured to engage the intramedullary canal's surrounding bone and enhance anchoring the tibia stem component 110J within the intramedullary canal. In the illustrated example shown in FIGS. 9D-9F, the plurality of surface features on the distal surface 131J are teeth having a biased orientation. Each distal surface 131J has two teeth as shown.

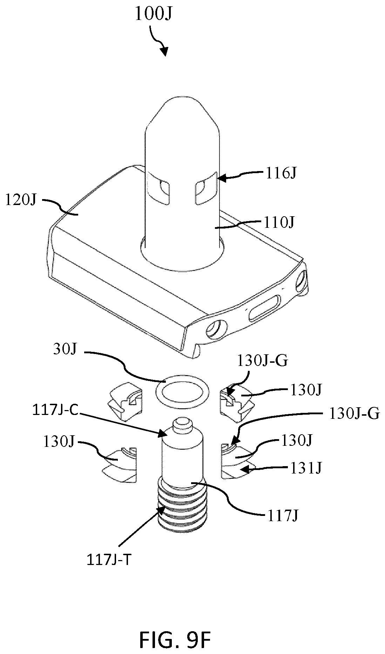

The two or more retractable members 130J are held together by an elastic ring 30J, such as a rubber band. (See FIGS. 9E-9F). Each of the two or more retractable members 130J are configured with a groove 130J-G (See FIG. 9F) for receiving the elastic ring 30J. The elastic ring 30J pulls the two or more retractable members 130J toward the axial center of the tibia stem component 110J and keep the retractable members 130J, particularly the cam surface 132J of the retractable members 130J, in contact with the cam engaging surface 117J-C.

In some embodiments of the prosthesis, the prosthesis can be structured similar to the embodiments 100C, 100D, 100E, 100F, 100G, 100H, except that rather than having the linear actuators 117C, 117D, 117E, 117G, and 117H that engage the one or more retractable members, an actuator (not shown) such as a rod or a stick can be inserted into the internal cavity 115C, ... 115H via the opening 123C, . . . 123H at the trailing end 112C, . . . 112H of the tibia stem to engage and move the one or more retractable members. In such embodiments, the leading tips of the actuator would be configured similar to the linear actuators 117C, 117D, 117E, 117G, and 117H to appropriately engage the cam surfaces 135C, 135D, 135E, 135G, and 135H of the one or more retractable members.

Referring to FIGS. 10A-10F, ankle prostheses 200A, 300A according to other embodiments of the present disclosure are disclosed. The prosthesis 200A, 300A comprises: a tibia stem component 210A, 310A and a tibia tray component 220A, 320A. The tibial stem component 210A, 310A comprises a leading end 211A, 311A, a trailing end 212A, 312A and a longitudinal axis L defined therethrough. The tibia tray component 220A, 320A is generally configured to be attached to a prosthetic joint articulating surface. The tibia tray component 220A, 320A extends from the trailing end 212A, 312A of the tibia stem component 210A, 310A. The tibia stem component 210A, 310A is sized and configured to be disposed in an intramedullary canal formed in a tibia. The tibia stem component 210A, 310A is divided into two or more retractable members 230A, 330A by longitudinal slits 231A, 331A formed in the tibia stem. The longitudinal slits 231A, 331A extend from the leading end 211A, 311A of the tibia stem component and partially toward the trailing end 212A, 312A, thereby defining each of the two or more retractable members 230A, 330A between a pair of the longitudinal slits 231A, 331A. The two or more retractable members 230A, 330A have proximal ends that are joined near the trailing end 212A, 312A of the tibia stem component and distal ends 233A, 333A that are freely moveable at the leading end 211A, 311A. The two or more retractable members 230A, 330A are configured to be controllably movable from a retracted position (shown in FIGS. 10A, 10B, and 10D) and have their free distal ends 233A, 333A at the leading end 211A, 311A be bent outward and away from the longitudinal axis L. In their retracted position, the two or more retractable members 230A, 330A are parallel to the longitudinal axis L and do not extend outward. When the tibia stem component 210A, 310A is disposed in the intramedullary canal and the two or more retractable members 230A, 330A are moved outward away from the longitudinal axis L, the two or more retractable members engage the intramedullary canal's surrounding bone and enhance anchoring the tibia stem component 210A, 310A within the intramedullary canal.

Referring to FIGS. 10B-10F, the two or more retractable members 230A, 330A define an internal cavity 215A, 315A that is open at both the leading end 211A, 311A of the tibia stem and the trailing end 212A, 312A of the tibia stem. The opening 213A, 313A at the trailing end 212A, 312A is accessible from the bottom side of the tibial tray 220A, 320A as shown in FIG. 10B and 10F. The tibia stem 210A, 310A further comprises a linear actuator 217A, 317A provided within the internal cavity 215A, 315A. The linear actuator 217A, 317A is configured to be movable within the internal cavity 215A, 315A along the longitudinal axis L, whereby the two or more retractable members 230A, 330A are moved from the retracted position and extend outward by moving the linear actuator 217A, 317A within the internal cavity 215A, 315A.

In some embodiments, the linear actuator 217A, 317A and the internal cavity's sidewall threadedly engage each other for moving the linear actuator 217A, 317A within the internal cavity 215A, 315A. The translation of the linear actuator 217A, 317A toward the trailing end 212A, 312A of the tibia stem component enables the two or more retractable members 230A, 330A, specifically the distal ends 233A, 333A, to extend outward away from the longitudinal axis L.

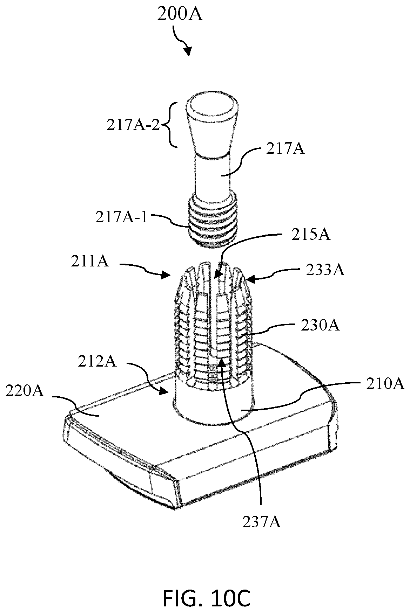

In some embodiments, the linear actuator 217A, 317A comprises a leading end and a trailing end corresponding with the direction of the tibia stem's leading end 211A, 311A and trailing end 212A, 312A, and further comprises a cam surface 217A-2, 317A-2 near its leading end. The side of each of the one or more retractable members 230A, 330A facing the internal cavity 215A, 315A engage the cam surface of the linear actuator 217A, 317A and will be referred to herein as the "cam engaging surface" 235A, 335A. (See FIG. 10B, 10G). The cam surface 217A-2, 317A-2 and the cam engaging surface 235A, 335A are configured to push the two or more retractable members 230A, 330A outward and away from longitudinal axis L when the linear actuator 217A, 317A translates toward the trailing end 212A, 312A of the tibia stem component. As can be seen in FIGS. 10B and 10C, the cam surface 217A-2, 317A-2 portion of the linear actuator 217A, 317A has an inverse frusto-conical shape so that the diameter of the distal tip of the cam surface portion is greater than the diameter of the internal cavity 215A, 315A formed by the cam engaging surfaces 235A, 335A of the two or more retractable members 230A, 330A. Thus, as the linear actuator 217A, 317A is translated downward in the orientation shown in FIG. 10B, 10G toward the trailing end 212A, 312A of the tibia stem component 210A, 320A, the cam surface portion 217A-2, 317A-2 pushes the two or more retractable members 230A, 330A outward away from the longitudinal axis L, effectively making the outside diameter of the leading end 211A, 311A of the tibia stem component larger. The distal tip 233A, 333A of the two or more retractable members 230A, 330A will move further outward as the linear actuator 217A, 317A is translated further toward the trailing end 212A, 312A. Thus, this controlled outward movement of the two or more retractable members 230A, 330A can be used to control the amount of force the two or more retractable members 230A, 330A are applying against the surrounding bone inside the intramedullary canal to secure the prosthesis inside the intramedullary canal.

In the tibia stem embodiment 200A, the linear actuator 217A comprises a threaded portion 217A-1 on its outer surface (see FIG. 10C) and the sidewall of the internal cavity 215A comprises a threaded portion 213A-1 (see FIG. 10B). Engagement of these screw threaded surfaces enables translation of the linear actuator 217A. By turning the linear actuator 217A clockwise or counter-clockwise, the linear actuator 217A can be moved up or down within the internal cavity 215A by operation of their threaded engagement. The linear actuator 217A can be provided with a tool-receiving socket 217A-S on the bottom end (the end near the trailing end 212A) that is accessible through the opening 213A (See FIG. 10B) at the bottom of the tibial tray portion 220A so that a tool such as a wrench, a screwdriver, etc. can be used to turn the linear actuator 217A.

Referring to FIGS. 10F-10H, in the tibia stem embodiment 300A, the linear actuator 317A is configured with a threaded hole 317A-1 open to its bottom end for receiving a threaded bolt 318A. The threaded bolt 318A is inserted into the threaded hole 317A-1 via a hole 313A at the bottom of the tibial tray portion 320A. Engagement of these screw threaded surfaces enables translation of the linear actuator 317A. By turning the threaded bolt 318A clockwise or counter-clockwise, the linear actuator 317A can be moved up or down by operation of their threaded engagement. The linear actuator 317A can be provided with a tool-receiving socket 317A-S on the bottom end that is accessible through the opening 313A (See FIG. 10G) at the bottom of the tibial tray portion 320A so that a tool such as a wrench, a screwdriver, etc. can be used to turn the threaded bolt 318A.

Preferably, to prevent the linear actuator 317A from turning when the threaded bolt 318A is being threaded into the linear actuator 317A, the linear actuator 317A can be provided with one or more boss 317A-3 that protrudes from the exterior of the linear actuator 317A. Each of the one or more boss 317A-3 extends between two retractable members 330A and prevents the linear actuator 317A from rotating about its longitudinal axis when the threaded bolt 318A is being turned. In other words, the one or more boss 317A-3 provides counter-torque to the linear actuator 317A. The boss 317A-3 can take a variety of shapes such as one or more ridges, splines, edges, grooves, etc. that meshes with the two or more retractable members 330A.

In some embodiments, the two or more retractable members 230A, 330A can comprise a plurality of surface features 237A that are configured to enhance the engagement with the intramedullary canal's surrounding bone and enhance anchoring the tibia stem component 210A, 310A within the intramedullary canal when the tibia stem component is disposed in the intramedullary canal and the two or more retractable members 230A, 330A are moved outward to engage the surrounding bone.

In some embodiments, the surface features 237A can be surface texturing or teeth having a biased orientation. For example, in the illustrated embodiment shown in FIGS. 10A-10C, the outer surface of the two or more retractable members 230A are provided with a plurality of teeth having a biased orientation that will resist the tibia stem component from being pulled out of the intramedullary canal. once the two or more retractable members 230A are deployed outward and the plurality of surface features 237A engage the surrounding bone in the intramedullary canal.

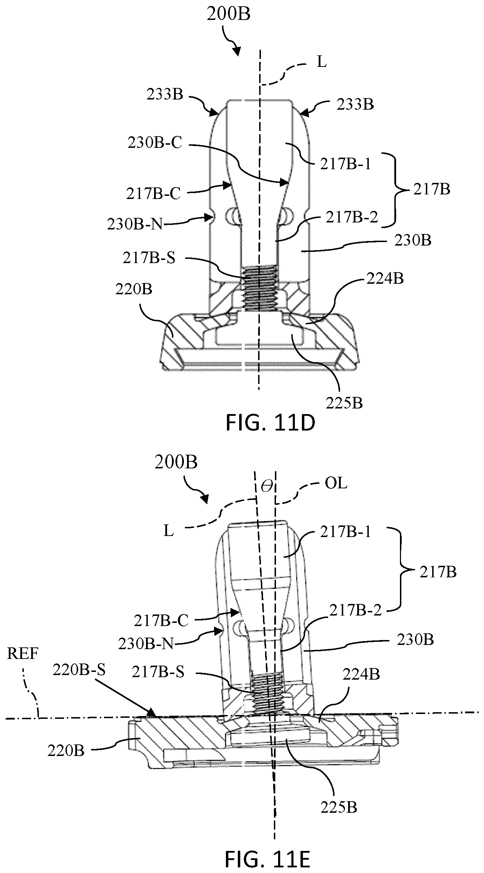

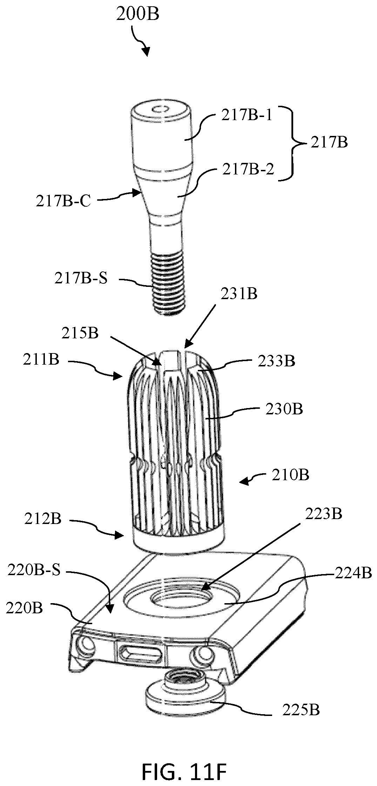

Referring to FIGS. 11A-11F, according to other embodiments of the present disclosure, disclosed is an ankle prosthesis 200B comprising: a tibia stem component 210B and a tibia tray component 220B. The tibial stem component 210B comprises a leading end 211B, a trailing end 212B, and a longitudinal axis L defined therethrough. The tibia tray component 220B is generally configured to be attached to a prosthetic joint articulating surface. The tibia tray component 220B extends from the trailing end 212B of the tibia stem component 210B. The tibia stem component 210B is sized and configured to be disposed in an intramedullary canal formed in a tibia. The tibia stem component 210B is divided into two or more retractable members 230B by longitudinal slits 231B (see FIGS. 11A, 11F) formed in the tibia stem component 210B. The longitudinal slits 231B extend from the leading end 211B of the tibia stem component and partially toward the trailing end 212B, thereby defining each of the two or more retractable members 230B between a pair of the longitudinal slits 231B. The two or more retractable members 230B have proximal ends that are joined near the trailing end 212B of the tibia stem component and distal ends 233B (see FIGS. 11D, 11F) that are freely moveable at the leading end 211B. The two or more retractable members 230B are configured to be controllably movable from a retracted position (shown in FIGS. 11A and 11B) to an extended position in which their free distal ends 233B at the leading end 211B be bent outward and away from the longitudinal axis L. In their retracted position, the two or more retractable members 230B are parallel to the longitudinal axis L and do not extend outward. When the tibia stem component 210B is disposed in the intramedullary canal and the two or more retractable members 230B are moved outward away from the longitudinal axis L, the two or more retractable members engage the intramedullary canal's surrounding bone and enhance anchoring the tibia stem component 210B within the intramedullary canal.

The two or more retractable members 230B define an internal cavity 215B that is open at both the leading end 211B of the tibia stem component and the trailing end 212B of the tibia stem. The internal cavity 215B can be best seen in FIG. 11F. The tibia stem component 210B further comprises a linear actuator 217B provided within the internal cavity 215B. The linear actuator 217B is configured to be movable within the internal cavity 215B along the longitudinal axis L, whereby the two or more retractable members 230B are moved from the retracted position and extend outward by moving the linear actuator 217B within the internal cavity 215B.

Referring to FIGS. 11D-11E, similar to the linear actuator 217A in the prosthesis 200A, the linear actuator 217B comprises a first portion 217B-1 that has a larger diameter than the remaining second portion 217B-2 and a transition portion in between that forms a cam surface 217B-C. The linear actuator 217B is situated inside the internal cavity 215B so that the first portion 217B-1 is at the leading end 211B (see FIG. 11B for the notation) of the tibia stem component. The interior sides of the two or more retractable members 230B that are facing into the internal cavity 215B are configured so that when the linear actuator 217B is translated downward toward the trailing end 216B of the tibia stem component 210B, the large diameter first portion 217B-1 interacts with the two or more retractable members 230B to push them outward and away from the longitudinal axis L.

In some embodiments, the contour of the interior sides of the two or more retractable members 230B are configured so that the diameter of the internal cavity 215B matches the reducing diameter of the linear actuator 217B going from the leading end 211B to the trailing end 212B. Thus, the interior surface of each of the two or more retractable members 230B comprise a cam engaging surface 230B-C that correspond to the cam surface 217B-C. This configuration is illustrated in FIGS. 11D-11E. Then, starting from the retracted configuration for the two or more retractable members 230B shown in FIGS. 11D-11E, when the linear actuator 217B is translated downward toward the trailing end 212B, the cam surface 217B-C and the cam engaging surface 230B-C operate on one another to push the two or more retractable members 230B outward and away from the longitudinal axis L.

The distal tip 233B of the two or more retractable members 230B will move further outward as the linear actuator 217B is translated further toward the trailing end 212B. Thus, this controlled outward movement of the two or more retractable members 230B can be used to control the amount of force the two or more retractable members 230B are applying against the surrounding bone inside the intramedullary canal to secure the prosthesis inside the intramedullary canal.

Referring to FIGS. 11A-11E, because the lower portion (i.e., closer to the trailing end 212B) of the two or more retractable members 230B becomes thicker as the diameter of the internal cavity 215B becomes smaller, this increase in thickness can hinder the ability for the retractable members 230B to flex or bend outward. This can be especially true if the two or more retractable members 230B are made of stiff material. Therefore, in some embodiments, each of the two or more retractable members 230B can be provided with one or more thinner necked regions 230B-N to tune the flexibility of the retractable members 230B to achieve either plastic or elastic deformation that would be suitable to the material, size, and anatomical application. Such necked regions 230B-N can be created by cutouts on the exterior and/or interior sides of the retractable members 230B.

The translation of the linear actuator 217B is achieved by the use of a base nut 225B. (See FIGS. 11D, 11E, and 11F). The tibia tray component 220B in this embodiment comprises a hole 223B that aligns with the internal cavity 215B so that the threaded portion 217B-S of the linear actuator 217B can be extended downward and through the hole 223B. The base nut 225B is threaded onto the threaded portion 217B-S from the bottom side of the tibia tray component 220B. Surrounding the hole 223B is a flange 224B (See FIG. 11F) and by tightening the base nut 225B the flange 224B is captured between the base nut 225B and the tibia stem component 210B. Thus, with the base nut 225B braced against the flange 224B, turning the base nut 225B and engaging the threaded portion 217B-S will pull the linear actuator 217B downward toward the trailing end 211B. This downward motion causes the stem component 210b to expand circumferentially, applying a hoop-like stress to the surrounding bone and embedding the implant. In one embodiment, the retractable members 230B are configured and arranged to bend within their elastic deformation range for the implant material of choice. Keeping the design within the elastic deformation range allows the retractable members 230B to spring back to the original position and allow for easy implant removal out of bone forming the intramedullary canal.

### Polyaxial Feature

According to some embodiments, the tibial stem component and the tibial tray component of the ankle prosthesis can be configured to be modular pieces so that the angle between the two components can be adjusted to be within a range of angles rather than being fixed to one configuration. Some examples of such ankle prosthesis are disclosed with the accompanying illustrations in FIGS. 11A-12I.

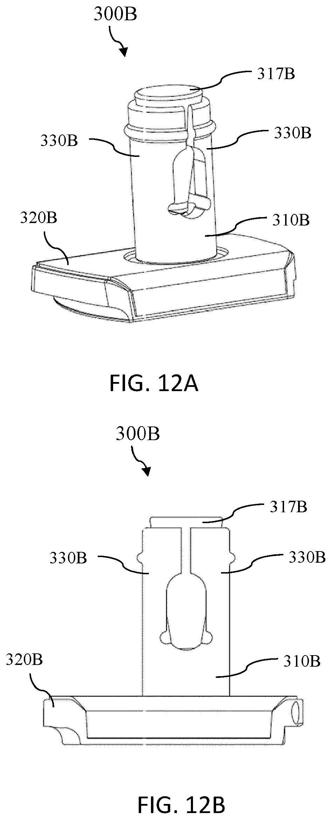

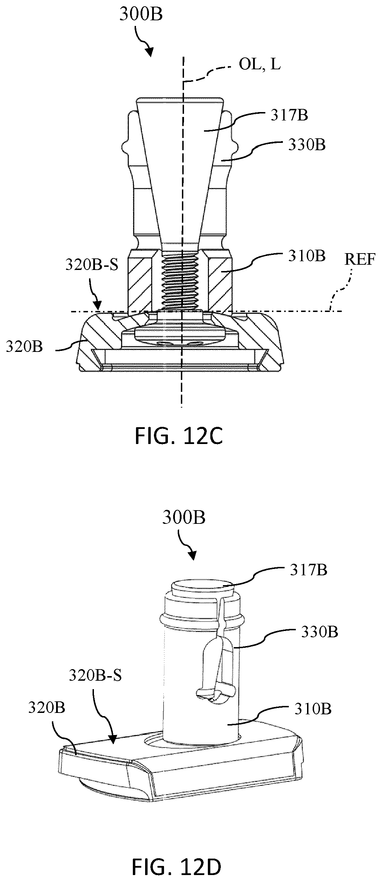

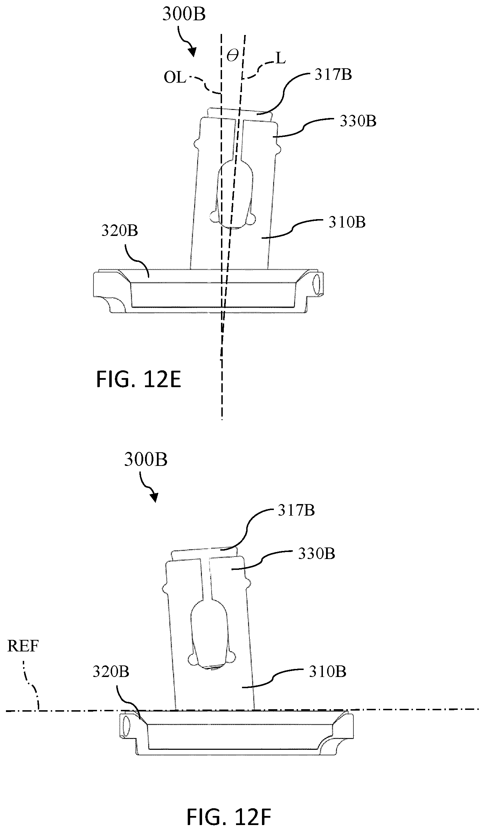

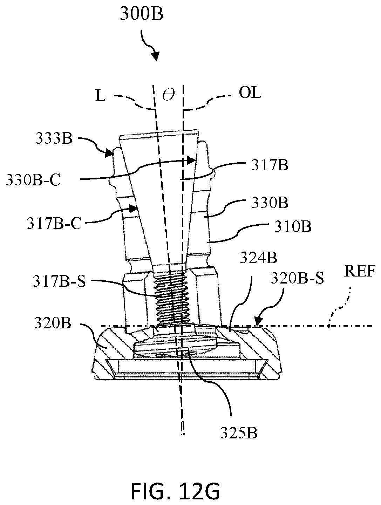

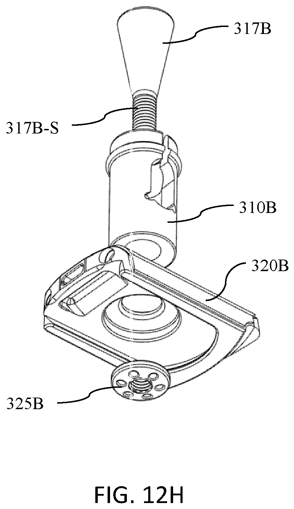

In some embodiments, the ankle prosthesis 200B, 300B comprises a tibia stem component 210B, 310B and a tibia tray component 220B, 320B. The tibia stem component comprises a leading end 211B, 311B, a trailing end 212B, 312B, and a longitudinal axis L defined therethrough. The tibia tray component 220B, 320B comprises a surface 220B-S, 320B-S defining a reference plane REF (see FIG. 11E, 12F, 12G). The tibia stem component and the tibia tray component are modular and the tibia tray component 220B, 320B and the trailing end of the tibia stem component 210B, 310B are configured to form a joint wherein the tibia stem component's relative angle with respect to the reference plane REF can be adjusted so that the longitudinal axis L of the tibia stem component 210B, 310B forms an angle *θ* (see FIG. 11E) with an orthogonal OL of the reference plane REF, wherein the angle *θ* can be between 0 degrees up to and including 45 degrees.

In one embodiment, the angle *θ* can range between 0 degrees up to and including 40 degrees. In one embodiment, the angle *θ* can range between 0 degrees up to and including 30 degrees. In one embodiment, the preferred angle *θ* can range between 0 degrees up to and including 20 degrees. In other joints of the body, this angular adjustability may be adjusted between 0 and 90 degrees, and as is suitable to the anatomy. The angular range is configured and arranged to suit the implant's size as well as anatomical considerations. In one embodiment, not shown, the angular range may be limited by flat faces in the apparatus's ball joint which limit angular motion in one plane differently than in another plane. This includes a uni-planar adjustable design, which may allow for more angular adjustment in the anterior-posterior direction (sagittal plane) while limiting angular motion in medial lateral direction (coronal). The direction of adjustability may be adapted to meet patient needs, such as providing a uni-planar design that pivots medial-lateral instead of anterior-posterior. It is understood that the adjustability may be further limited to only medial tile away from the perpendicular or longitudinal axis. The exact plane of uni-planar adjustability may be adjusted to suit the patient needs wherein any plane of motion can be controlled which pivots about the longitudinal axis.

The tibia stem component 210B, 310B is sized and configured to be disposed in an intramedullary canal formed in a tibia. The tibia stem component comprises: two or more retractable members 230B, 330B, configured to be controllably movable from a retracted position and be extended outward and away from the longitudinal axis L. **In** the retracted position, the two or more retractable members 230B, 330B are contained substantially within the silhouette of the tibia stem component and do not extend outward. When the tibia stem component is disposed in the intramedullary canal and the one or more retractable members 230B, 330B are extended outward, the one or more retractable members engage the intramedullary canal's surrounding bone and enhance anchoring the tibia stem within the intramedullary canal.

The adjustable joint formed between the tibial stem component 210B, 310B and the tibial tray component 220B, 320B can be secured to fix the angle *θ* at a desired angle between the above-mentioned range between 0 degrees up to and including 45 degrees. The tibia tray component 220B, 320B is configured with a recess 224B, 324B on the top surface 220B-S, 320B-S of the tibia tray component 220B, 320B for receiving the trailing end 212B, 312B of the tibia stem component 210B, 310B. The bottom of the recess 224B, 324B is configured with a convex surface to receive the trailing end 212B, 312B of the tibial stem component 210B, 310B and allow the tibia stem component 210B, 310B to swivel against the convex surface to adjust the angle *θ* to a desired angle.

Once the desired angle *θ* is achieved, the position of the tibia stem component 210B, 310B can be locked by threading the locking nut 225B, 325B onto the threaded portion 217B-S, 317B-S of the linear actuator 217B, 317B. The locking nut 225B, 325B captures the flange 224B, 324B between the locking nut 225B, 325B and the tibia stem component 210B, 310B and tightening the locking nut 225B, 325B locks the assembly's configuration. In some embodiments, supplemental locking features can be used in conjunction with the locking nut 225B, 325B to enhance the locking function. Such supplemental locking features can be in the form of nylon washers, locking washers, distorted thread nuts, serrated face nuts, or other locking nut/washer combinations. In some embodiments, Spiralock^{®} thread forms by Stanley^{®} Engineering Fastening can be used.

This polyaxial adjustment feature allows the prosthesis to be adjusted to the variations in the patients' physiology during the ankle prosthesis implantation surgical procedure.

### Some material Considerations

In some embodiments, the tibia stem components 210A, 310A, and 210B of the prostheses 200A, 310A, and 200B, respectively, can be made of a memory metal alloys such as Nitinol so that the superelastic properties or the memory properties of such alloy can be employed to enhance the function of the two or more retractable members 230A, 330A, and 230B.

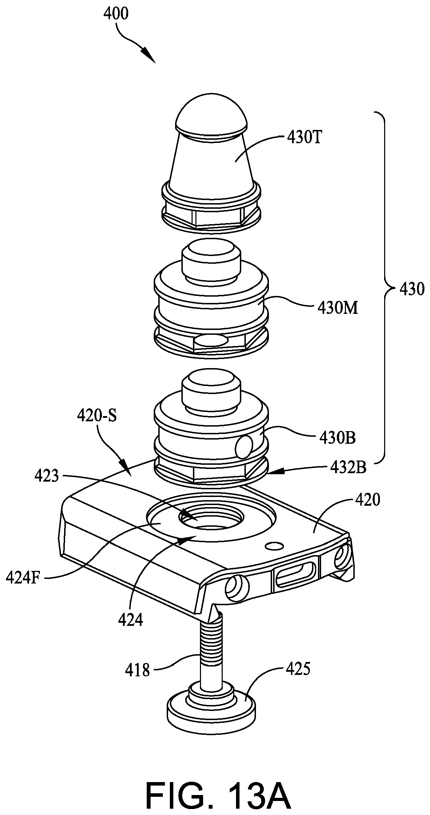

The polyaxial adjustment feature can also be applied on tibia stem prostheses that comprise stackable modular tibia stem components, for example, the tibia stem components in Wright Medical's Inbone^{™} Total Ankle System. An example of such tibia stem prosthesis 400 is illustrated in FIGS. 13A-13B. In some embodiments, the ankle prosthesis 400 comprises a tibia stem component 430 and a tibia tray component 420. The tibia stem component can comprise two or more modular stem pieces that are configured to be stacked and securely engage to each other. The two or more modular stem pieces can include a base stem component 430B, a top stem component 430T, and one or more middle stem component 430M. The modular stem pieces can be threaded into each other. The modular configuration of the tibia stem 430 allows the surgeon to configure different lengths for the tibia stem based on the condition and dimension of the patient's tibia. The diameter of the modular stem pieces can be varied also.

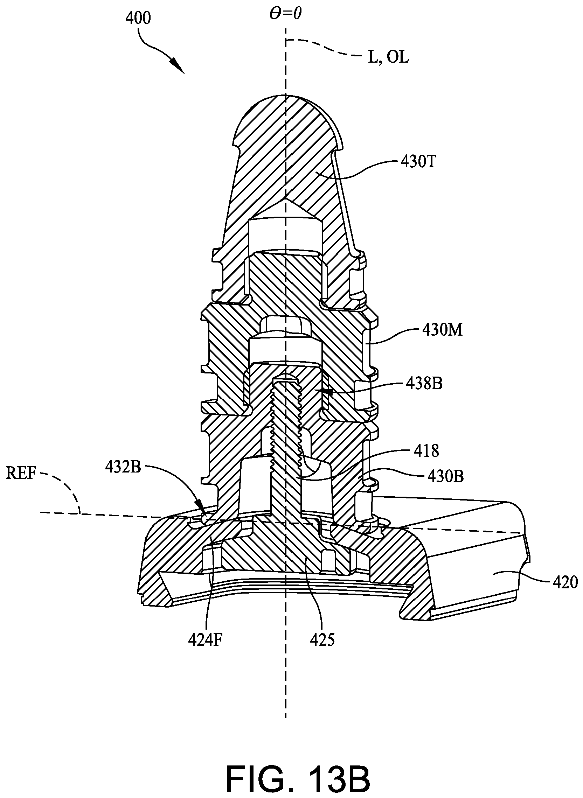

In the illustrated example, the tibia stem component 430 includes three modular pieces: the base stem component 430B, the middle stem component 430M, and the top stem component 430T. The base stem component 430B is configured to form a joint wherein the base stem component's relative angle with respect to the reference plane REF can be adjusted so that the longitudinal axis L of the base stem component 430B forms an angle *θ* (see FIG. 13B) with an orthogonal OL of the reference plane REF, wherein the angle *θ* can be between 0 degrees up to and including 45 degrees. In the configuration illustrated in FIG. 13B, the angle *θ* is 0. The reference plane REF is defined by a surface 420-S that is on the proximal side (in the orientation of the tibia tray 420 as installed on to the prepared distal end of the patient's tibia) of the tibia tray component 420.

In one embodiment, the angle *θ* can range between 0 degrees up to and including 40 degrees. In one embodiment, the angle *θ* can range between 0 degrees up to and including 30 degrees. In one embodiment, the preferred angle *θ* can range between 0 degrees up to and including 20 degrees. In other joints of the body, this angular adjustability may be adjusted between 0 and 90 degrees, and as is suitable to the anatomy. The angular range is configured and arranged to suit the implant's size as well as anatomical considerations. In one embodiment, not shown, the angular range may be limited by flat faces in the apparatus's ball joint which limit angular motion in one plane differently than in another plane. This includes a uni-planar adjustable design which may allow for more angular adjustment in the anterior-posterior direction (sagittal plane) while limiting angular motion in medial lateral direction (coronal).

The adjustable joint formed between the base stem component 430B and the tibia tray component 420 can be secured to fix the angle *θ* at a desired angle between the above-mentioned range between 0 degrees up to and including 45 degrees. The tibia tray component 420 is configured with a recess 424 on the top surface 420-S of the tibia tray component 420 for receiving a trailing end 432B of the base stem component 430B. The bottom of the recess 424 is configured with a convex surface to receive the trailing end 432B of the base stem component 430B and allow the assembled tibia stem component 430 to swivel against the convex surface to adjust the angle *θ* to a desired angle. The convex surface at the bottom of the recess 424 is convex in the proximal direction.

Once the desired angle *θ* is achieved, the position of the tibia stem component 430 can be locked by threading a locking bolt 425 into the base stem component 430B from the distal side of the tibia tray 420 through a hole 423 that is provided in the convex surface of the recess 424. The convex surface forms an annular flange 424F around the hole 423. The locking bolt 425 comprises a head portion and a threaded shaft portion 418. The threaded shaft portion 418 has a male-type threads and the base stem component 430B is configured with a corresponding female type threaded hole 438B for receiving the threaded shaft portion 418.

As shown in the longitudinal cross-sectional view in FIG. 13B, the head portion of the locking bolt 425 is larger than the diameter of the hole 423 and captures the flange 424F between the head portion of the locking bolt 425 and the base stem component 430B and tightening the locking bolt 425 locks the assembly's configuration. The locking bolt 425 threadedly engages the trailing end of the tibial stem component 430 from the distal surface of the tibia tray to lock the joint at the angle *θ*.

In some embodiments, supplemental locking features can be used in conjunction with the locking bolt 425 to enhance the locking function. Such supplemental locking features can be in the form of nylon washers, locking washers, distorted thread nuts, serrated face nuts, or other locking nut/washer combinations. In some embodiments, Spiralock^{®} thread forms by Stanley^{®} Engineering Fastening can be used.

In some embodiments, the multiaxial modular stems and implants may include one or more counter-torque features. The one or more counter-torque features advantageously facilitate the tightening of the modular stems by resisting unwanted rotation as the stems or implants are assembled.

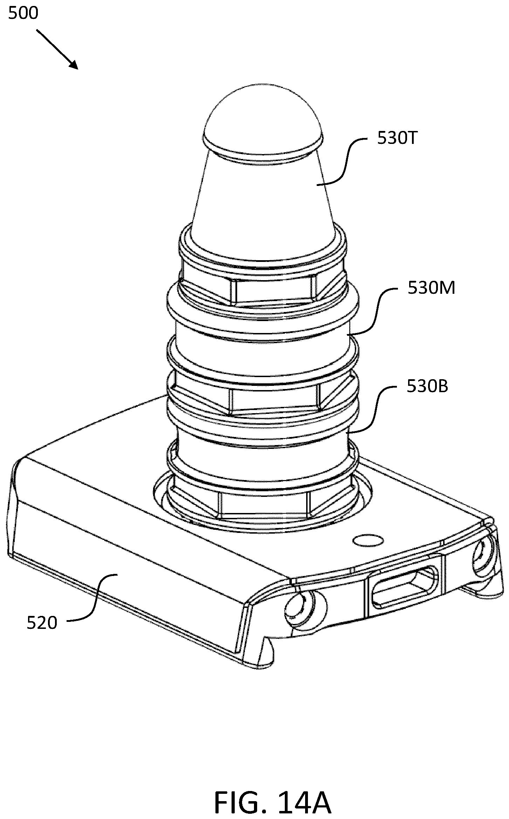

FIGS. 14A-14C illustrate one example of a prosthesis that includes one or more counter-torque features. The prosthesis 500 illustrated in FIGS. 14A-14C is similar to the prosthesis 400 illustrated in FIGS. 13A-13B. Components or features of the prosthesis 500 that are similar or the same as the components or features of the prosthesis 400 have the same reference numerals increased by "100" and duplicative descriptions are not repeated.

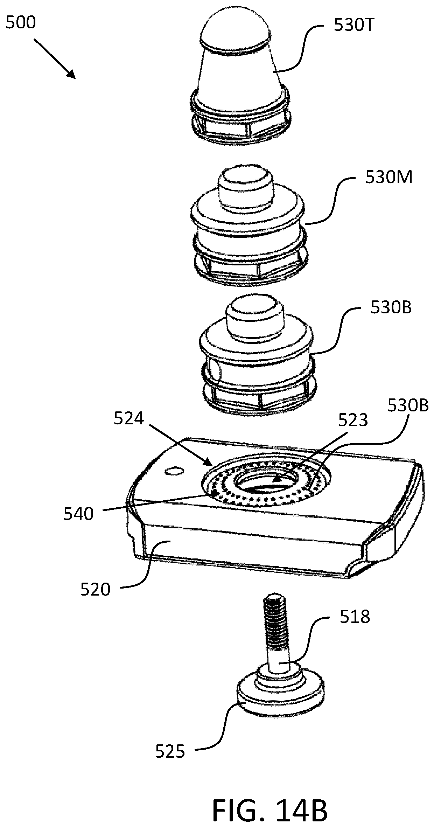

As best seen in FIG. 14B, the tibial tray component 520 may include one or more first counter-torque features 540 that surrounds the hole 523 within the recess 524. The one or more first counter-torque features 540 are shown in FIG. 14A as a series of bumps that arranged around the hole 523, but it should be understood that the one or more counter-torque features 540 may take one or more of a variety of forms, including other types of projections (e.g., beads, spikes, teeth), knurling, grooves, or other surface texturing to increase the friction between the tibial tray component 520 and the base stem component 530B. In another embodiment not shown, the counter-torque features 540 may cover a convex dome 539, which protrudes above the top surface of the tibial tray component 520. In other words, it is not required that the dome and counter-torque features 540 are recessed relative to the tibial tray component 520.

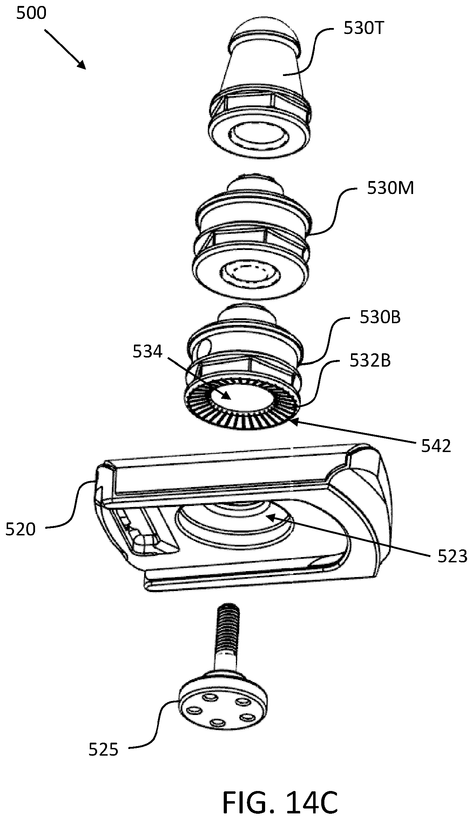

As best seen in FIG. 14C, the trailing end 532B of the base stem component 530B may include one or more second counter-torque features 542. The one or more second counter-torque features 542 as shown in FIG. 14C as a series of alternating ridges and troughs that radially extend outwardly from hole 534. In some embodiments, the one or more second counter-torque features 542 may be sized and configured to engage the one or more first counter-torque features 524. As will be understood by one of ordinary skill in the art, the increased friction between the tibial tray component 520 and the base stem component 530B due to the presence of one or both of the one or more first and second counter-torque features 540, 542 will counter the torque applied to the base stem component 530B when the locking bolt 525 is tightened to lock the stem 530 to the tibial tray component 520.

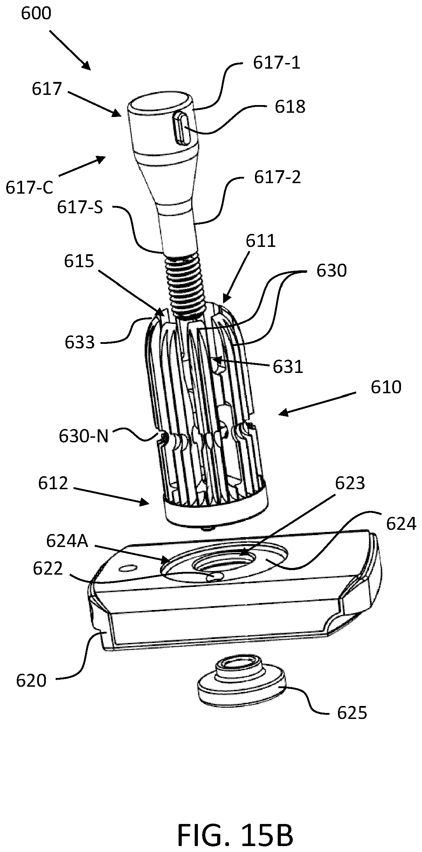

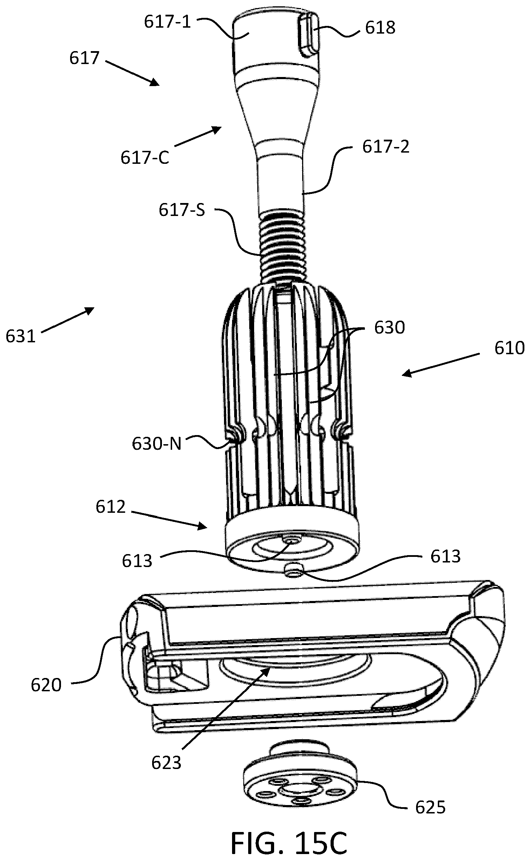

FIGS. 15A-15D illustrate an embodiment of a modular prosthesis in accordance with the invention. The modular prosthesis 600 includes a tibia stem component 610 and a tibia tray component 620. As shown in FIGS. 15B and 15C, the tibial stem component 610 includes a leading end 611 and a trailing end 612. The tibial stem component 610 defines a longitudinal axis.

The tibia tray component 620 may be configured to be attached to a prosthetic joint articulating surface. The tibia tray component 620 may extend from the trailing end 612 of the tibia stem component 610. The tibia stem component 610 may be sized and configured to be disposed in an intramedullary canal formed in a tibia. The tibia stem component 610 may be divided into two or more retractable members 630 by longitudinal slits 631 formed in the tibia stem component 610. The longitudinal slits 631 may extend from the leading end 611 of the tibia stem component 610 toward the trailing end 612 such that a retractable member 630 may be disposed between two longitudinal slits 631. The retractable members 630 may have proximal ends that are joined near the trailing end 612 of the tibia stem component 610 and distal ends 633 that may be freely moveable at the leading end 611.

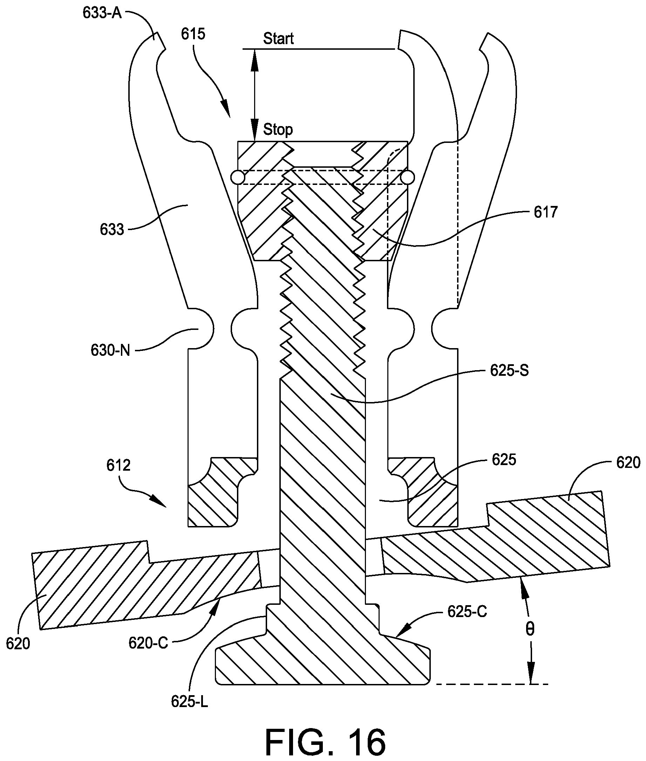

Although the distal ends 633 are shown as being straight, it should be understood that the distal ends 633 may include one or more barbs, projections, or other configurations 633A for retaining the linear actuator 617 within the cavity 615 as best seen in FIG. 16. The barbs or projections 633A may be dimensions such that they allow for the linear actuator 617 to be inserted into the cavity 615 when the retractable members 630 are outwardly flexed, but prevent the linear actuator 617 from being removed from the cavity 615 when the retractable members 630 are in their relaxed, normal position.

The retractable members 630 may be configured to be controllably movable from a retracted position to an extended position in which their free distal ends 633 at the leading end 611 be bent outward and away from the longitudinal axis. In the retracted position, the retractable members 630 may be parallel to the longitudinal axis and therefore may not extend outwardly. The tibia stem component 610 may be configured such that when the tibia stem component 610 is disposed in the intramedullary canal, the retractable members 630 may be moved outwardly away from the longitudinal axis and the retractable members 630 may engage the intramedullary canal's surrounding bone thereby enhancing the anchoring of the tibia stem component 610 within the intramedullary canal.

The retractable members 630 may define an internal cavity 615 that is open at both the leading end 611 of the tibia stem component 610 and the trailing end 612 of the tibia stem component 610. The tibia stem component 610 may include a linear actuator 617 provided within the internal cavity 615. The linear actuator 617 may be configured to be movable within the internal cavity 615, such as along the longitudinal axis, to move one or more of the retractable members 630 from the retracted position and to an extend position.

The linear actuator 617 may include a first portion 617-1, which may be a head portion, and a second portion 617-2, which may be a shaft portion. The first portion 617-1 may have a diameter or width that is greater than a diameter or width of the second portion 617-2. A transition portion 617-C may be disposed between and separate the first portion 617-1 and the second portion 617-2. The transition portion 617-C may form a cam surface. The linear actuator 617 may be disposed inside the internal cavity 615 so that the first portion 617-1 of the linear actuator 617 may be at the leading end 611 of the tibia stem component 610.

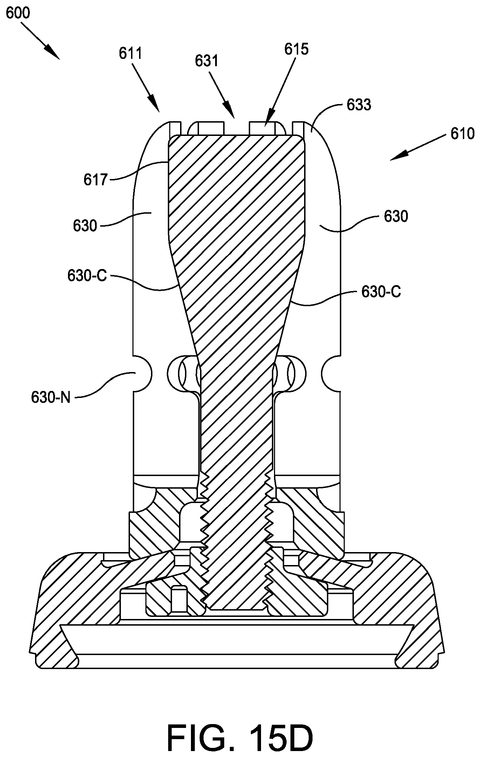

The interior sides of the retractable members 630 (e.g., the sides that are facing into the internal cavity 615) may be configured so that when the linear actuator 617 is translated downward toward the trailing end 612 of the tibia stem component 610, the cam surface 617-C and/or first portion 617-1 may interact with the retractable members 630 and push them outwardly and away from the longitudinal axis of the stem component 610. For example, the contour of the interior sides of the retractable members 630 may be configured so that the diameter of the internal cavity 615 matches the reducing diameter of the linear actuator 617 going from the leading end 611 to the trailing end 612. As best seen in FIG. 15D, the interior surface of one or more of the retractable members 630 comprise a cam engaging surface 630-C that correspond to the cam surface 617-C. In some embodiments, all of the retractable members 630 may include a cam engaging surface 630-C. When fewer than all retractable members 630 are provided with a cam engaging surface 630-C, the retractable members 630 without the cam-engaging surface may not be moved when the linear actuator 617 is translated within the internal cavity 615 such that the expansion of the retractable members 630 with a cam-engaging surface 630 may be directional.

Referring again to FIG. 15B, the distal tips 633 of the retractable members 630 may move farther outward as the linear actuator 617 is translated toward the trailing end 612. This controlled outward movement of the retractable members 630 can be used to control the amount of force the retractable member(s) 630 apply against the surrounding bone inside the intramedullary canal to secure the tibia stem component 610 inside the intramedullary canal.

In some embodiments, the lower portion (i.e., the portion closer to the trailing end 612) of the retractable members 630 may become thicker as the diameter of the internal cavity 615 becomes smaller. The increase in thickness can hinder the ability for the retractable members 630 to flex or bend outward, which can be especially true if the two or more retractable members 630 are made of stiff material. Therefore, in some embodiments, each of the retractable members 630 may include one or more thinner necked regions 630-N to tune the flexibility of the retractable member 630 to achieve either plastic or elastic deformation that would be suitable to the material, size, and anatomical application. Such necked regions 630-N may be created by cutouts on the exterior and/or interior sides of the retractable members 630.

The translation of the linear actuator 617 may be achieved by the use of a base nut 625, which is best seen in FIGS. 15B and 15C. In some embodiments, the tibia tray component 620 may include a hole 623 that aligns with the internal cavity 615 so that the shaft 617-S, which may include a threaded portion, of the linear actuator 617 can be extended downward and through the hole 623. The base nut 625 may be threaded onto the threaded portion of the shaft 617-S from the bottom side of the tibia tray component 620. The base nut 625 may include a contoured surface 625-S, which may be configured to engage a complementary surface 620-S of the tibia tray component 620. Note that although the base nut 625 is shown with a stepped shoulder or ledge 625-L, such feature may be omitted. The contoured surface 625-S of base nut 625 and corresponding complementary surface 620-S of tibia component 620 may be corresponding semi-spherical surfaces such that the stem component 610 may coupled to the tibia tray component 620 at an oblique or perpendicular angle. However, it should be understood that the surfaces 625-S, 620-S may have other geometries to facilitate locking at oblique angles. The contoured surfaces 625-S and 620-S may include one or more surface textures to facilitate locking. Examples of such surface textures include, but are not limited to, bead blasting, grit blasting, machining, polishing, and knurling, to list only a few possibilities.

A flange 624 may be disposed within a recess 624A surround the hole 623, and by tightening the base nut 625, the flange 624 may be captured between the base nut 625 and the tibia stem component 610. With the base nut 625 braced against the flange 624, turning the base nut 625 and engaging the threaded portion of the shaft portion 617-S of the linear actuator 617 will pull the linear actuator 617 downward toward the trailing end 611. This downward motion causes the stem component 610 to expand circumferentially, applying a hoop-like stress to the surrounding bone and embedding the implant. In one embodiment, the retractable members 630 may be configured and arranged to bend within their elastic deformation range for the implant material of choice. Keeping the design within the elastic deformation range allows the retractable members 630 to spring back to the original position and allow for easy implant removal out of bone forming the intramedullary canal.

The modular prosthesis 600 includes one or more counter-torque features. In some embodiments, a counter-torque feature may be provided on one or both of the linear actuator 617 and the tibia stem component 610, and another counter torque feature may be provided on one or both of the tibia stem component 610 and the tibia tray component 620. For example, the linear actuator 617 may include one or more protrusions 618, which may be sized and configured to be received within one or more longitudinal slits 631 defined between retractable members 630 of the tibia stem component 610 as seen in FIGS. 15A-15C. The one or more protrusions 618 may be configured to slide within respective slits 631 when the linear actuator 617 is moved in the internal cavity 615 when the base nut 625 is rotated. The engagement between the one or more protrusions 618 and the retractable members 630 when the one or more protrusions are disposed within the slits 631 prevents the linear actuator 617 from rotating when the base nut 625 is rotated.

The trailing end 612 of the tibia stem component 610 may include one or more pins or detents 613 that may extend from the trailing end 612 as best seen in FIG. 15C. The pins or detents 613 may have a cylindrical shape as shown or other shapes or structures. The pins or detents 613 may be sized and configured to be received in corresponding holes or recesses 622 defined by the tibia tray component 620. In some embodiments, the holes or recesses 622 may be defined by the flange 624 within recess 624A and adjacent to the hole 623 as best seen in FIG. 15B. The combination of the pins or detents 516 and corresponding holes or recesses 622 may be configured to resist or prevent the tibia stem component 610 from rotating relative to the tibia tray component 620 when the base nut 625 engages the linear actuator 617.

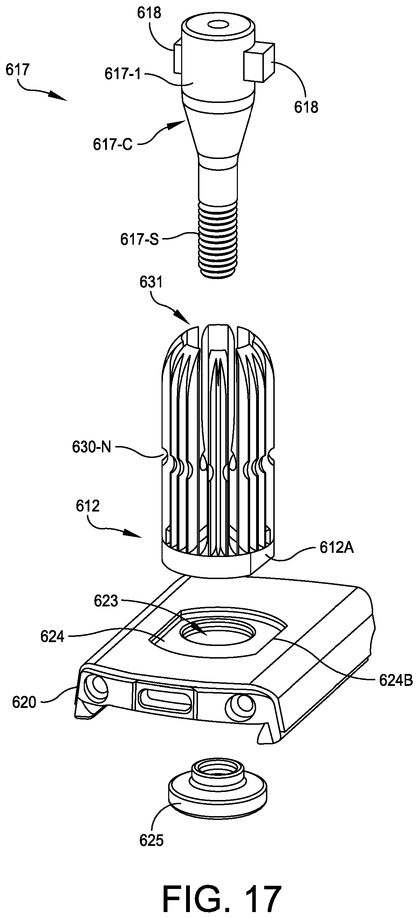

It should be understood that various counter-torque features may be used. For example, in the example illustrated in FIG. 17, the trailing end 612 of the tibia stem component 610 may include one or more flats 612A, which may be sized and configured to engage one or more flats 624B provided along the walls defining the recess 624A in which the flange 624 may be disposed as shown in FIG. 17. The engagement between the one or more flats 612A of the tibia stem component 610 and the one or more flats 624B of the tibia tray component 620 prevents the tibia stem component 610 from rotating relative to tibia tray component 620 when the base nut engages the linear actuator 625.

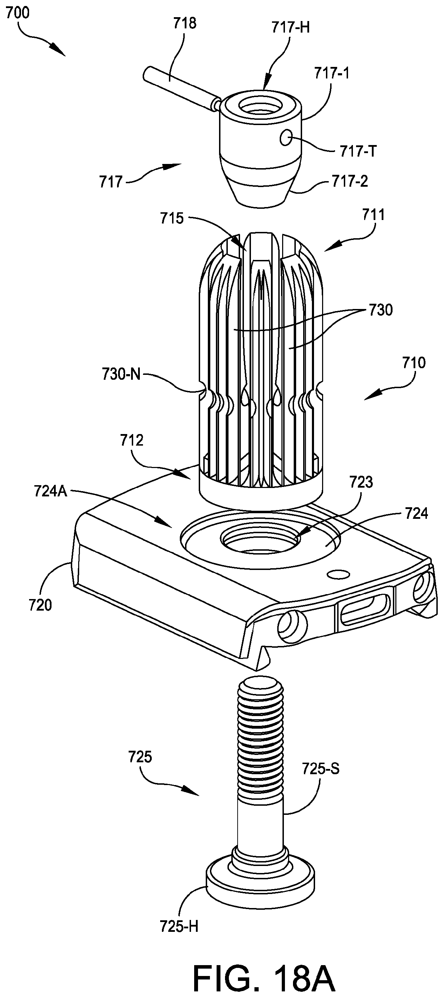

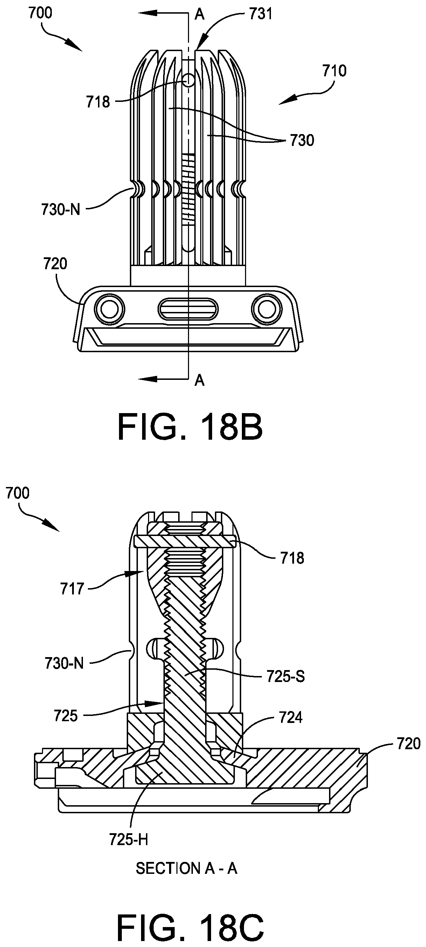

It should also be understood that various types of linear actuators may be used. For example, FIGS. 18A-18C illustrate another example of a modular implant or prosthesis 700. Implant 700 may include a tibia stem component 710 and a tibia tray component 720. The tibial stem component 10 may be sized and configured to be disposed in an intramedullary canal in a tibia. Tibia stem component 710 may include a leading end 711 and a trailing end 712 that may be coupled to the tibia tray component 720. The tibial stem component 710 may define a longitudinal axis extending from the leading end 711 to the trailing end 712.

The tibia stem component 710 may be divided into two or more retractable members 730 by longitudinal slits 731. The longitudinal slits 731 may extend from the leading end 711 of the tibia stem component 710 toward the trailing end 712 such that a retractable member 730 may be disposed between two longitudinal slits 731. The retractable members 730 may have proximal ends that are joined near the trailing end 712 of the tibia stem component 710 and distal ends 733 that may be freely moveable at the leading end 711. The retractable members 730 may be configured to be controllably movable from a retracted position to an extended position in which their free distal ends 733 at the leading end 711 be bent outward and away from the longitudinal. In the retracted position, the retractable members 730 may be parallel to the longitudinal axis and therefore may not extend outwardly. The tibia stem component 710 may be configured such that when the tibia stem component 710 is disposed in the intramedullary canal, the retractable members 730 may be moved outwardly away from the longitudinal axis and the retractable members 730 may engage the intramedullary canal's surrounding bone thereby enhancing the anchoring of the tibia stem component 710 within the intramedullary canal. The retractable members 730 may define an internal cavity 715 that is open at both the leading end 711 of the tibia stem component 710 and the trailing end 712 of the tibia stem component 710.

A linear actuator 717 may be provided within the internal cavity 715. The linear actuator 717 may be configured to be movable within the internal cavity 715, such as along the longitudinal axis, and to move one or more of the retractable members 730 from the retracted position and to an extended position. The linear actuator 717 may include a first portion 717-1, which may have a generally cylindrical shape, and a second portion 717-2, which may taper to a narrower diameter as it extends away from the first portion 717-1. In some embodiments, the second portion 717-2 may define a cam surface for engaging a complementary surface on one or more retractable members 730. Linear actuator 717 may define a threaded hole 717-H that extends through the actuator 717. In some embodiments, hole 717-H may be a blind hole defined by an end adjacent to the second portion 717-2. A transverse hole 717-T may be defined by the first portion 717-1 of the actuator 717. The transverse hole 717-T may intersect the threaded hole 717-H and be sized and configured to receive a pin 718 therein. The pin 718 may be dimensioned such it has a length that is greater than a diameter of the actuator 717 such that when the pin 718 is disposed within the transverse hole 717-T it may extend outwardly from the actuator 717 and may be at least partially received within one or more slits 631 defined by the tibia stem component 710.

Threaded hole 717-H may be sized and configured to be engaged by a bolt 725, which may be provided instead of a base nut. Bolt 725 may include an enlarged head 725-H and a shaft 725-S that may be at least partially threaded. Shaft 725-S may be dimensioned such that it extends through tibia tray component 720 and into the cavity 715 defined by the tibia stem component 710 where it is received within the threaded hole 717-H defined by the actuator 717. For example, the shaft 725-S of bolt 725 may be sized and configured to be received in hole 723 defined by the tibia tray component 720 that may be aligned with the internal cavity 715 of the tibia stem component 710.

The translation of the linear actuator 717 may be achieved by rotating the bolt 725 to engage the threaded portion of the shaft 725-S and the threaded hole 717-H of the actuator 717. Pin 718, which may form a counter-torque feature, is disposed within at least one slit 731 defined between adjacent retractable members 730 and resists and/or prevents the actuator 717 from rotating within the cavity 715. In addition to providing a counter-torque feature, pin 718 may also provide a stop for bolt 725 within the hole 717-H. Although not shown in FIGS. 18A-18C, the tibia stem component 710 and/or tibia tray component 720 may include one or more additional counter-torque features. For example, the tibia tray component 720 may include a series of bumps, similar to bumps 540 shown in FIG. 14B, surrounding the hole 723 on flange 724 within the recess 724A and/or tibia stem component 710 may include a series of alternating ridges and troughs similar to those shown in FIG. 14C. However, it should be understood that other types of counter-torque features may be provided on tibia stem component 7710 and/or tibia tray component 720.

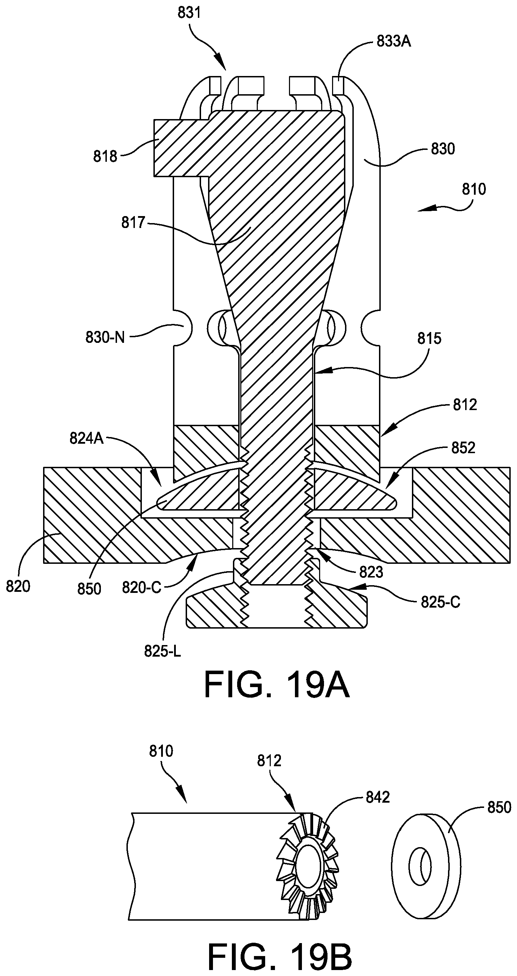

FIGS. 19A-19B illustrate another example of a modular prosthesis in accordance with some embodiments. The prosthesis 800 may include a tibia stem component 810 and a tibia tray component 820. The tibia stem component 810 may define a longitudinal axis extending from a leading end 811 to a trailing end 812. The tibia stem component 810 may be divided into two or more retractable members 830 by longitudinal slits 831, which may extend from the leading end 811 of the tibia stem component 810 toward the trailing end 812 such that a retractable member 830 may be disposed between two longitudinal slits 831. The retractable members 830 may have proximal ends that are joined near the trailing end 812 of the tibia stem component 810 and distal ends 833 that may be freely moveable at the leading end 811. The tibia stem component 810 may define a cavity 815 in which a linear actuator 817 is disposed. The trailing end 812 of the stem component 810 may have a curved surface to facilitate angulation of the tibia stem component relative to the tibia tray component 820. In some embodiments, trailing end 812 may include one or more counter-torque features 842, such as splines, grooves, troughs, knurling, texturing, or any other suitable counter-torque feature as will be understood by one of ordinary skill in the art.

Tibia tray component 820 may define a hole 823 located within a recess 824A. Recess 824A may be sized and configured to receive, at least partially, the trailing end trailing end 812 of the tibia stem component 810. The flange 824 may have a substantially planar upper surface (e.g., the surface closest to the tibia stem component 810), although flange 824 may have a curved or otherwise contoured upper surface similar to the other flanges disclosed herein. Recess 824A may also be sized and configured to receive a washer 850.

Washer 850 may be a "crushable" or "deformable" washer to facilitate locking of the tibia stem component 810 at a desired angle. For example, washer 850 may be formed from a material that is softer than a material of the trailing end 812 of the tibia stem component 810 and the tibia tray component 820. In one non-limiting example, the tibia stem component 810, or at least a trailing end 812 of the tibia stem component 810, and the tibia tray component 820 may be formed from a titanium alloy and the washer 850 may be formed from pure titanium. One of ordinary skill in the art will understand that other materials may be used for the tibia stem component 810, tibia tray component 820, and washer 850. Washer 850 may include a curved upper surface 852 that may be configured to facilitate coupling the tibia stem component 810 to the tibia tray component 820 at a user-selectable angle. The lower surface 854 of washer 850 may be planar, although it should be understood that it may have other shapes, including a curved shape. It should further be understood that the crushable washer 850 may be used in connection with other implants/prosthesis disclosed herein.

Further, in a manner similar to that described above with respect to FIG. 16, the base nut 825 may include a contoured surface 825-S, which may be configured to engage a complementary surface 820-S of the tibia tray component 820, as shown in FIG. 19A. Note that although the base nut 825 is shown with a stepped shoulder or ledge 825-L, such feature may be omitted. The contoured surface 825-S of base nut 825 and corresponding complementary surface 820-S of tibia component 820 may be corresponding semi-spherical surfaces such that the stem component 810 may coupled to the tibia tray component 820 at an oblique or perpendicular angle. However, it should be understood that the surfaces 825-S, 820-S may have other geometries to facilitate locking at oblique angles. The contoured surfaces 825-S and 820-S may include one or more surface textures to facilitate locking. Examples of such surface textures include, but are not limited to, bead blasting, grit blasting, machining, polishing, and knurling, to list only a few possibilities.

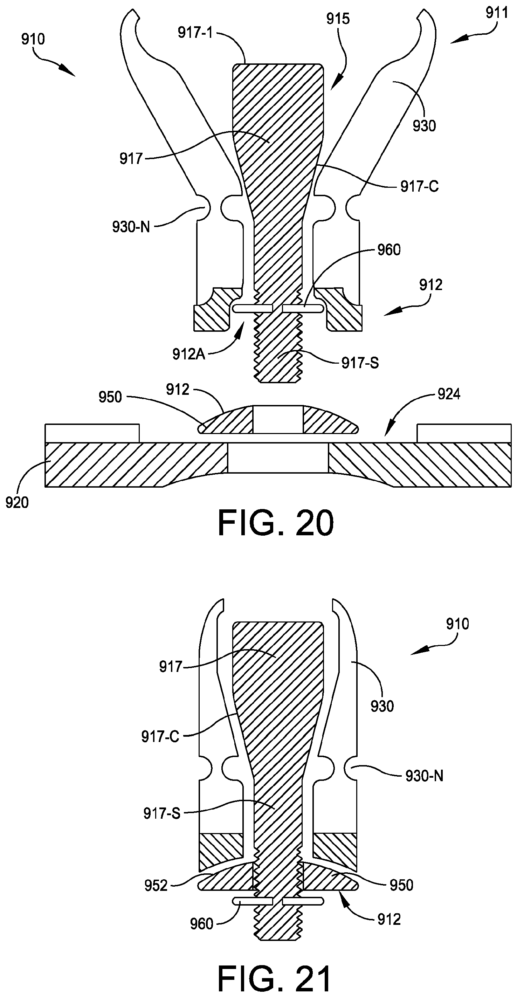

FIG. 20 illustrates another example of an implant or prosthesis in accordance with some embodiments. The implant 900 illustrated in FIG. 20 may include a tibia stem component 910, a tibia tray component 920, a crushable washer 950, and a securing member 960 for securing a linear actuator 917 within a cavity 915 of the tibia stem component 910.

Tibia stem component 910 may define a longitudinal axis extending from a leading end 911 to a trailing end 912. Similar to the other tibia stem components described herein, the tibia stem component 910 may be divided into two or more retractable members 930 that are configured to expand upon insertion and/or advancement of the linear actuator 917. The tibia stem component 910 may define an internal cavity 915 that is sized and configured to receive linear actuator 917 in a similar manner to the way other linear actuators described herein are received within the cavity to facilitate expansion of the retractable members in response to advancement of the linear actuator 917. The trailing end 912 of tibia stem component 910 may define a recessed region 912A that is sized and configured to receive the securing member 960.

In the example illustrated in FIG. 20, the securing member 960 is shown as being a split ring, but one of ordinary skill in the art will understand that the securing member 960 may take other forms, such as a nut, cross-pin, or deformation, to list only a few possibilities. The securing member 960 may be configured to engage the linear actuator 917, such as along a length of the stem portion 917-S, and the trailing end 912 of the tibia stem component 910 to maintain the linear actuator 917 within the cavity 915. For example, the securing member 960 may engage the threads or a recess disposed along the length of the stem 917-S and may have an outer diameter that engages the surfaces defining the recessed region 912A.

Tibia tray component 920 may define a hole 923 located within a recess 924A. Recess 924A may be sized and configured to receive, at least partially, the trailing end trailing end 912 of the tibia stem component 910. The flange 924 may have a substantially planar upper surface (e.g., the surface closest to the tibia stem component 910), although flange 924 may have a curved or otherwise contoured upper surface similar to the other flanges disclosed herein. Recess 924A may be sized and configured to receive a washer 950.

Like washer 850 described above, washer 950 may be a "crushable" or "deformable" washer to facilitate locking of the tibia stem component 910 at a desired angle. In some embodiments, washer 950 may be formed from a material that is softer than a material of the trailing end 912 of the tibia stem component 910 and the tibia tray component 920. For example, the tibia stem component 910, or at least a trailing end 912 of the tibia stem component 910, and the tibia tray component 920 may be formed from a titanium alloy and the washer 950 may be formed from pure titanium. It should be understood that other materials may be used for the tibia stem component 910, tibia tray component 920, and washer 950. Washer 950 may include a curved upper surface 952 that may be configured to facilitate coupling the tibia stem component 910 to the tibia tray component 920 at a user-selectable angle. The lower surface 954 of washer 950 may be planar, although it should be understood that it may have other shapes, including a curved shape. A base nut (not shown) may be provided to engage the linear actuator 917, as described above.

In some embodiments, such as the example illustrated in FIG. 21, the securing member may be configured to maintain engagement between the tibia stem component 910, linear actuator 917, and the washer 950. As shown in FIG. 21, the washer 950 is disposed along the length of the shaft 917-S of the linear actuator 917 and the securing member 960 may secure the washer 950 to the linear actuator 917 and may secure the linear actuator 917 within the cavity 915 of the tibia stem component 910.

As noted above, an implant may be provided with a stem component that is configured to expand in a selectable direction. In some embodiments, the direction may be selectable intraoperatively by a surgeon. It should be understood that the selection of the direction may be intraoperatively, but also may be made taking into account preoperatively obtained information (e.g., consulting one or more preoperatively acquired images).

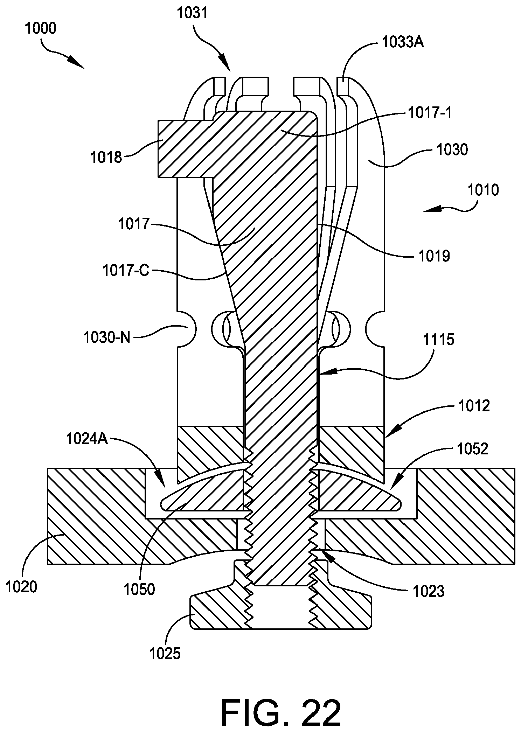

FIG. 22 illustrates one example of a modular prosthesis 1000 configured to provide directional engagement with surrounding bone. The prosthesis illustrated in FIG. 22 is similar to the prosthesis illustrated in FIG. 19A-19B with the same or similar features or components being identified with the same reference numeral increased by "200." Repetitive descriptions of like components are not provided. The modular prosthesis includes a linear actuator 1017 configured with a flat 1019 or other feature to provide clearance between the linear actuator 1017 and at least one of the one or more retractable members 1030. More specifically, the flat 1019 may be positioned along one side of the linear actuator and extend at least partially along the first portion 1017-1 and the transition portion 1017-C of the linear actuator 1017. In some embodiments, the flat 1019 may be located on an opposite side of the linear actuator 1017 as the protrusion 1018 as shown in FIG. 22, although flat 1019 may be positioned at other locations relative to the protrusion 1018, including on the same side such that the protrusion 1018 extends radially outwardly from the flat 1019. A surgeon or other medical professional or user may select the direction in which the one or more retractable members may be expanded by selecting the longitudinal slit 1031 into which the protrusion 1018 is inserted.

When the base nut 1025 is rotated such that the linear actuator 1017 is pulled axially toward the tibia tray component 1020, the protrusion slides within a longitudinal slit 1031 and the cam surface of the transition portion 1017-C engages the corresponding cam surface of at least one of the one or more retractable members 1030. Due to the presence of the 1019, at least one of the one or more retractable members 1030 is not engaged by the transition portion 1017-C and thus is not urged radially outward as the linear actuator is advanced distally. In this manner, only certain retractable members 1030 are urged radially outwardly to engage the surrounding bone while other retractable members 1030 remain in a relax or natural state. Such a configuration may be advantageous when the surrounding bone is of differing quality.

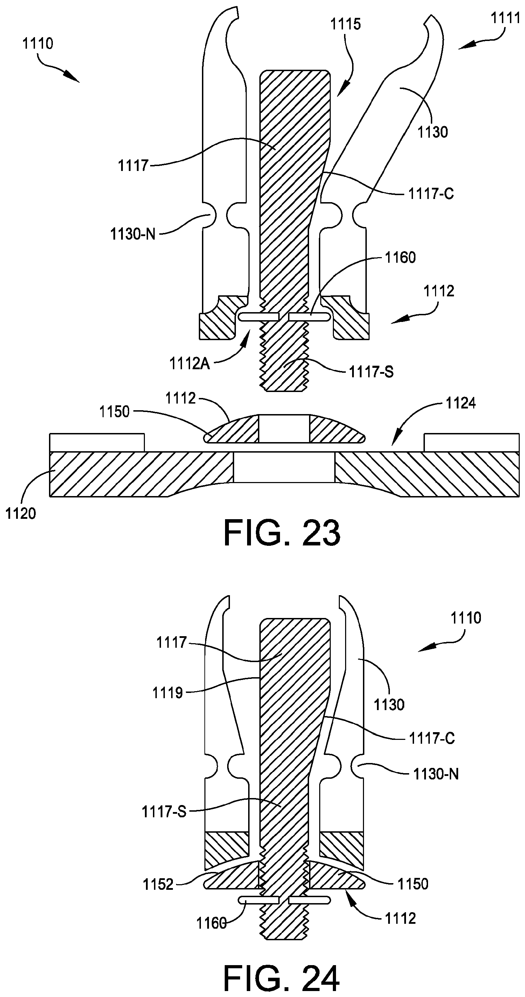

Although the linear actuator 1017 is described as including a flat 1019, it should be understood that the linear actuator 1017 may be configured such that it has a generally cylindrical shape along its entire length and includes a single cam surface (e.g., an angled protrusion or ramped surface), which may encircle less than half of the circumference of the linear actuator. For example, the single cam surface may encircle approximately 1/10, 1/9, 1/8, 1/7, 1/6, 1/5, 1/4, or 1/3 of the circumference of the linear actuator 1017. However, it should be understood that the single cam surface may encircle more than half of the circumference of the linear actuator 1017. FIGS. 23 and 24 illustrate examples of implants 1100 that are similar to those shown in FIGS. 20 and 21 described above. The features or components of the implants 1100 illustrated in FIGS. 23 and 24 that are the same or similar to the implants 900 described above with respect to FIGS. 20 and 21 have the same reference numeral increased by "200" and repetitive descriptions are not provided. The linear actuator 1117 shown in FIGS. 23 and 24 includes a flat portion 1119 that is similar to the flat portion 1019 described above with respect to FIG. 22.

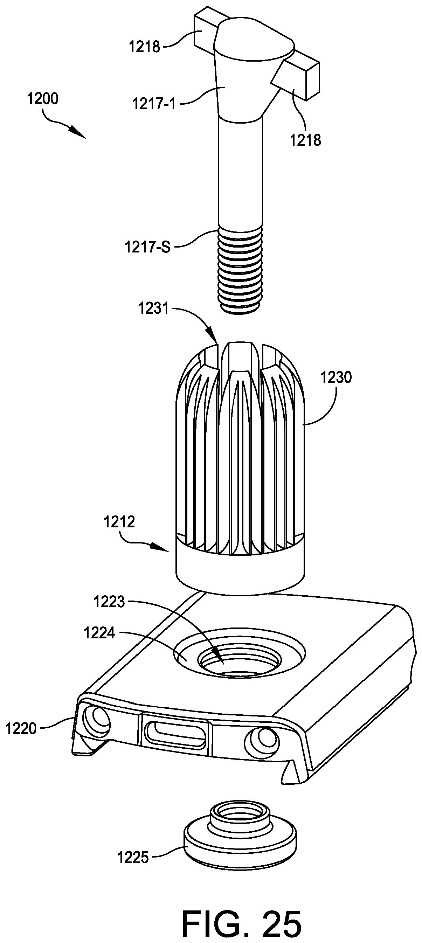

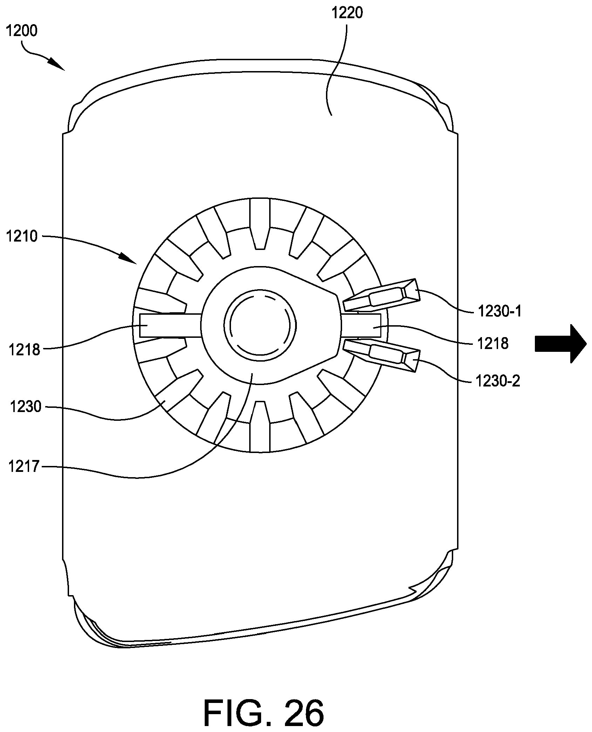

FIGS. 25 and 26 illustrate another example of an implant 1200 that includes a counter-torque feature and is configured to provide directional engagement with surrounding bone. The implant 1200 is similar to the implants 600 shown in FIGS. 15A-17 and like components or features have the same reference numeral increased by "600." Duplicative or repetitive descriptions are not provided. As shown in FIG. 25, the first portion 1217-1 of linear actuator 1217 includes a pair of outwardly extending protrusions 1218 that are sized and configured to be received within respective slits 1231 disposed between adjacent retractable members 1230. The first portion 1217-1 also includes a ramped surface 1217-C, which forms a cam surface, extending radially in one direction and encircle only a portion of the circumference of the first portion 1217-1. As best seen in FIG. 26, only certain retractable members, i.e., retractable members 1230-1 and 1230-2, are deflected outwardly in the direction indicated by the arrow when the linear actuator 1217 is moved linearly in a distal direction in response to the base nut 1225 being rotated.

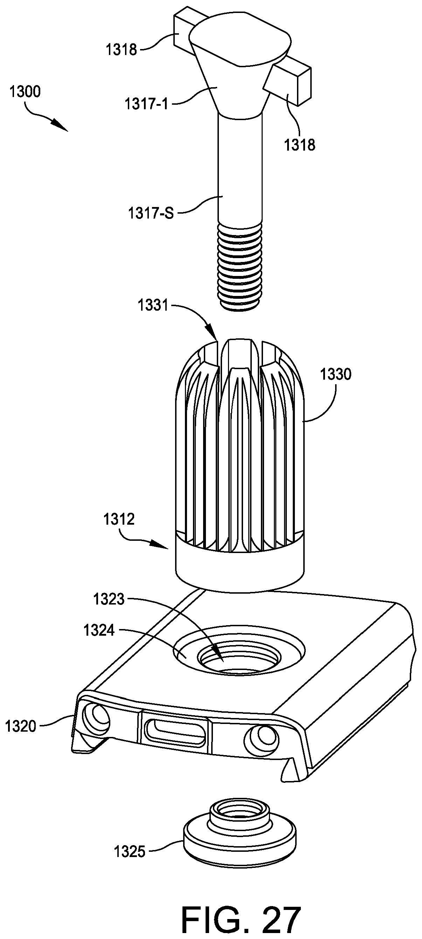

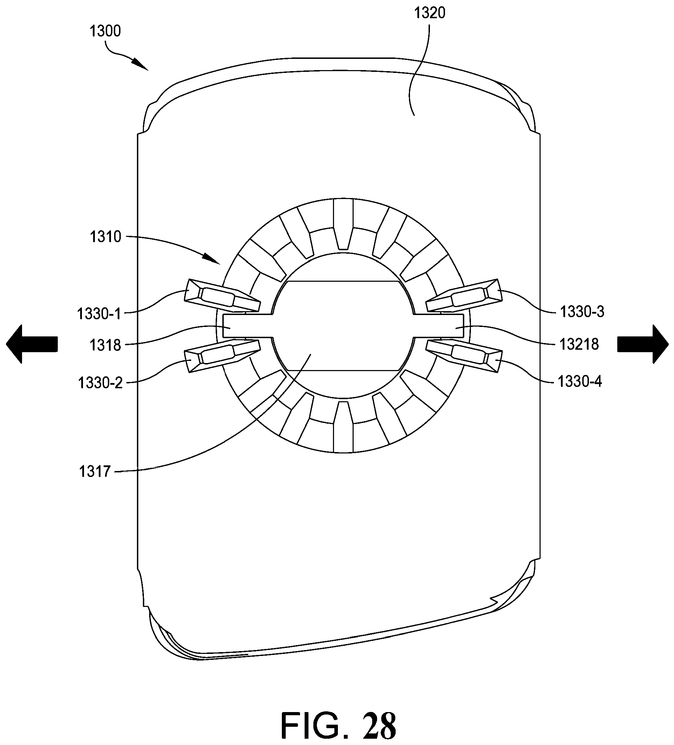

FIGS. 27 and 28 illustrate another example of an implant 1300 that includes a counter-torque feature and is configured to provide directional engagement with surrounding bone. The implant 1300 is similar to the implant 1200 shown above in FIGS. 26 and 27 and like components or features are designated with the same reference numeral increased by "100." Repetitive descriptions are not provided. The first portion 1317-1 of the linear actuator 1317 includes a pair of oppositely positioned cam surfaces 1317-C with a protrusion 1318 extend outwardly from each cam surface. As best seen in FIG. 28, each cam surface causes a pair of retractable members 1330 to extend outwardly as the linear actuator 1317 is moved distally in response to the rotation of the base nut 1325. For example, retractable members 1330-1, 1330-2 may be moved by a first cam surface and retractable members 1330-3, 1330-3 may be moved by a second cam surface that is disposed on the opposite side of the linear actuator 1317. The arraows in FIG. 28 identify the directions in which the retractable members are flexed. As will be appreciated from the foregoing description, the location and size of the cam surfaces of the linear actuator 1317 may be adjusted to provide the desired expansion.

The tibia stem component and/or the tibia tray component in the various embodiments of the prosthesis disclosed herein can be made of any total joint material of materials commonly used in the prosthetic art, including, but not limited to, metals, ceramics, titanium, titanium alloys, tantalum, chrome cobalt, surgical steel, polyethylene, absorbable polymer, or any other total joint replacement metal and/or ceramic. In some embodiments, the tibia stem component and/or the tibia tray component can comprise a coating of 3D printed Biofoam^{™}, Adaptis^{™}, porous metal, sintered glass, artificial bone, any uncemented metal or ceramic surface, or a combination thereof that would promote bony in-growth. The tibia stem component and/or the tibia tray component can further be covered with one or more coatings such as antimicrobial, antithrombotic, and osteoinductive agents, or a combination thereof. In embodiments where the above-mentioned porous coating is provided, these agents can further be carried in a biodegradable carrier material with which the pores in the porous coating can be impregnated.

Although the examples of the inventive structures illustrated herein are exemplified as tibia stem and tray components of an ankle replacement prosthesis, the inventive structures are equally applicable in other physical joints in human or animal skeleton.

It will be understood that the foregoing description is of exemplary embodiments of this invention, and that the invention is not limited to the specific forms shown. Modifications may be made in the design and arrangement of the elements without departing from the scope of the invention, which is defined by the claims.

## Claims

1. An ankle prosthesis (600), comprising:
a first component (620) defining a hole and configured to be attached to a prosthetic joint articulating surface, wherein the first component is a tibia tray component; and
a second component (610) configured to be coupled to the first component, wherein the second component is a tibia stem component, the second component including a leading end (611) and a trailing end (612), the second component defining an internal cavity (615) and including a retractable member (630), the retractable member configured to be moved from a first position to a second position intraoperatively; and
an actuator (617) configured to be received in and move along the internal cavity of the second component, the actuator including a first counter-torque feature (618) configured to engage the second component and to resist rotation of the actuator when the actuator is engaged by a third component.

2. The prosthesis of claim 1, wherein the third component includes a base nut (625), and wherein the actuator includes a threaded stem (617-S) that is configured to be engaged by the base nut.

3. The prosthesis of claim 2, wherein the actuator includes a cam surface (617-C) disposed adjacent to the threaded stem, the cam surface configured to engage an internal surface (630-C) of the retractable member, wherein typically the first counter-torque feature includes a protrusion extending from a head portion of the actuator that is disposed adjacent to the cam surface, and wherein the protrusion is configured to be received in a slit disposed adjacent to the retractable member.

4. The prosthesis of any of claims 2-3, further comprising a washer (850) sized and configured to be received between the first component and the second component and to receive the threaded stem, the washer configured to deform in response to a pressure applied by the first component and the second component.

5. The prosthesis of claim 4, further comprising a securing member (960) configured to secure the actuator within the internal cavity, wherein typically the securing member includes a split ring sized and configured to be disposed on the threaded stem of the actuator.

6. The prosthesis of any of the preceding claims, wherein the trailing end of the second component includes a second counter-torque feature (542), the second counter-torque feature configured to engage the first component and to resist rotation of the second component when the actuator is engaged by the third component.

7. The prosthesis of claim 6, wherein the second counter-torque feature includes surface texturing; or wherein the second counter-torque feature includes a protrusion that is configured to be received in a corresponding recess defined by the first component; or wherein the second counter-torque feature includes a flat surface formed on the trailing end of the second component that is configured to engage a corresponding surface on the first component; or wherein the second counter-torque feature includes a recess that is configured to receive a protrusion extending from a surface of the first component.

8. The prosthesis of any of the preceding claims, wherein the third component includes a threaded bolt, and wherein the actuator includes a body having a cam surface and a head portion, the body defining a threaded hole at one end that is configured to at least partially receive the threaded bolt.

9. The prosthesis of claim 8, wherein the first counter-torque feature includes a protrusion extending from the head portion, the protrusion sized and configured to be received in a slit disposed adjacent to the retractable member; or wherein the threaded hole is a blind hole; or wherein the threaded hole extends through the body of the actuator, the body defining a second hole that receives a pin having a length that is greater than a width of the body.

10. The prosthesis of any of the preceding claims, wherein a tip of the retractable member includes a projection configured to retain the actuator within the internal cavity.

11. The prosthesis of claim 1, wherein:
the stem component is sized and configured to be received in an intramedullary canal formed in a bone, the actuator being configured to move the retractable member from the first position to the second position; and
wherein the first counter-torque feature is configured to resist rotation of the stem component when the actuator is actuated.

12. The prosthesis of claim 11, wherein the retractable member
includes a projection configured to retain the actuator within the internal cavity;
or wherein the first counter-torque feature includes a surface texture at the trailing end of the stem component; or wherein the first counter-torque feature includes a protrusion extending from the actuator and disposed within a slit defined by the stem component; or wherein the first counter-torque feature includes a protrusion extending from the trailing end of the stem component; or wherein the first counter-torque feature includes a recess defined by the trailing end of the stem component, the recess sized and configured to receive a projection that extends from a surface of another component of the prosthesis;
or wherein the first counter-torque feature includes a flat formed at the trailing end of the stem component, the flat configured to be engaged by a complementary surface of another component of the prosthesis.

13. The prosthesis of claim 11, wherein the actuator includes:
a first portion;
a second portion including a threaded shaft that extends through a hole defined by the trailing end of the stem; and
a third portion disposed between the first portion and the second portion, the third portion including a cam surface, wherein a securing member is typically disposed along a length of the second portion and retains the actuator within the internal cavity.

14. The prosthesis of claim 13, wherein the securing member includes a split ring; or wherein the prosthesis further comprises a deformable washer disposed between the trailing end of the stem and the securing member; or wherein the first portion of the actuator includes a projection that is received within a slit disposed adjacent to the retractable member, wherein the trailing end of the stem component includes a second counter-torque feature (542), and wherein the second counter-torque feature includes a surface texture at the trailing end of the stem component.

15. The prosthesis of claim 14, wherein the second counter-torque feature includes a protrusion extending from the actuator and disposed within a slit defined by the stem component; or wherein the second counter-torque feature includes a protrusion extending from the trailing end of the stem component; or wherein the second counter-torque feature includes a recess defined by the trailing end of the stem component, the recess sized and configured to receive a projection that extends from a surface of another component of the prosthesis; or wherein the second counter-torque feature includes a flat formed at the trailing end of the stem component, the flat configured to be engaged by a complementary surface of another component of the prosthesis.

16. The prosthesis of claim 11, wherein the actuator includes a body having a first portion and a second portion, the second portion tapering from the first portion and providing a cam surface, the body defining a hole that is at least partially threaded at one end, wherein optionally the first counter-torque feature includes a protrusion extending from the first portion and received within a slit disposed adjacent to the retractable member.

17. The prosthesis of claim 16, wherein the hole defined by the body of the actuator extends through the body, the body defining a transverse hole in which a pin is disposed, the pin having a length that is greater than a width of the body such that at least a portion of the pin is received within a slit disposed adjacent to the retractable member, wherein the first counter-torque feature includes a surface texture at the trailing end of the stem component, or wherein the first counter-torque feature includes a protrusion extending from the trailing end of the stem component, or wherein the first counter-torque feature includes a recess defined by the trailing end of the stem component, the recess sized and configured to receive a projection that extends from a surface of another component of the prosthesis, or wherein the first counter-torque feature includes a flat formed at the trailing end of the stem component, the flat configured to be engaged by a complementary surface of another component of the prosthesis.

## Patentansprüche

1. Fußgelenkprothese (600), umfassend:
eine erste Komponente (620), die ein Loch definiert und zur Befestigung an einer artikulierenden Fläche einer Gelenkprothese konfiguriert ist, wobei die erste Komponente eine Tibiaschalenkomponente ist; und
eine zweite Komponente (610), die konfiguriert ist, um mit der ersten Komponente gekoppelt zu werden, wobei die zweite Komponente eine Tibiaschaftkomponente ist, die ein vorderes Ende (611) und ein hinteres Ende (612) aufweist, die zweite Komponente einen inneren Hohlraum (615) definiert und ein einziehbares Element (630) aufweist, wobei das einziehbare Element konfiguriert ist, um intraoperativ von einer ersten Position in eine zweite Position bewegt zu werden; und
einen Aktuator (617), der konfiguriert ist, um in dem inneren Hohlraum der zweiten Komponente aufgenommen zu werden und sich entlang diesem zu bewegen, wobei der Aktuator ein erstes Gegendrehmomentmerkmal (618) aufweist, das konfiguriert ist, um mit der zweiten Komponente in Eingriff zu treten und einer Drehung des Aktuators zu widerstehen, wenn der Aktuator mit einer dritten Komponente in Eingriff steht.

2. Prothese nach Anspruch 1, wobei die dritte Komponente eine Basismutter (625) aufweist, und wobei der Aktuator einen Gewindeschaft (617-S) aufweist, der konfiguriert ist, um mit der Basismutter in Eingriff zu treten.

3. Prothese nach Anspruch 2, wobei der Aktuator eine Nockenoberfläche (617-C) aufweist, die neben dem Gewindeschaft angeordnet ist, wobei die Nockenoberfläche konfiguriert ist, um mit einer Innenfläche (630-C) des einziehbaren Elements in Eingriff zu treten, wobei das erste Gegendrehmomentmerkmal üblicherweise einen Vorsprung aufweist, der sich von einem Kopfabschnitt des Aktuators erstreckt, der neben der Nockenoberfläche angeordnet ist, und wobei der Vorsprung konfiguriert ist, um in einem Schlitz aufgenommen zu werden, der neben dem einziehbaren Element angeordnet ist.

4. Prothese nach einem der Ansprüche 2 bis 3, ferner umfassend eine Unterlegscheibe (850), die dazu bemessen und konfiguriert ist, um zwischen der ersten Komponente und der zweiten Komponente aufgenommen zu werden und den Gewindeschaft aufzunehmen, wobei die Unterlegscheibe konfiguriert ist, um sich als Reaktion auf einen von der ersten Komponente und der zweiten Komponente ausgeübten Druck zu verformen.

5. Prothese nach Anspruch 4, ferner umfassend ein Sicherungselement (960), das konfiguriert ist, um den Aktuator im inneren Hohlraum zu sichern, wobei das Sicherungselement üblicherweise einen Spaltring aufweist, der dazu bemessen und konfiguriert ist, um auf dem Gewindeschaft des Aktuators angeordnet zu werden.

6. Prothese nach einem der vorstehenden Ansprüche, wobei das hintere Ende der zweiten Komponente ein zweites Gegendrehmomentmerkmal (542) aufweist, wobei das zweite Gegendrehmomentmerkmal konfiguriert ist, um mit der ersten Komponente in Eingriff zu treten und einer Drehung der zweiten Komponente zu widerstehen, wenn der Aktuator mit der dritten Komponente in Eingriff steht.

7. Prothese nach Anspruch 6, wobei das zweite Gegendrehmomentmerkmal eine Oberflächenstrukturierung einschließt; oder wobei das zweite Gegendrehmomentmerkmal einen Vorsprung aufweist, der konfiguriert ist, um in einer entsprechenden durch die erste Komponente definierten Aussparung aufgenommen zu werden, oder wobei das zweite Gegendrehmomentmerkmal eine flache Oberfläche aufweist, die am hinteren Ende der zweiten Komponente ausgebildet ist und konfiguriert ist, um mit einer entsprechenden Oberfläche an der ersten Komponente in Eingriff zu treten; oder wobei das zweite Gegendrehmomentmerkmal eine Aussparung aufweist, die konfiguriert ist, um einen Vorsprung aufzunehmen, der sich von einer Oberfläche der ersten Komponente erstreckt.

8. Prothese nach einem der vorstehenden Ansprüche, wobei die dritte Komponente einen Gewindebolzen aufweist, und wobei der Aktuator einen Körper mit einer Nockenoberfläche und einem Kopfabschnitt aufweist, wobei der Körper an einem Ende ein Gewindeloch definiert, das konfiguriert ist, um den Gewindebolzen zumindest teilweise aufzunehmen.

9. Prothese nach Anspruch 8, wobei das erste Gegendrehmomentmerkmal einen Vorsprung aufweist, der sich vom Kopfabschnitt erstreckt, wobei der Vorsprung dazu bemessen und konfiguriert ist, um in einem Schlitz aufgenommen zu werden, der neben dem einziehbaren Element angeordnet ist; oder wobei das Gewindeloch ein Sackloch ist; oder wobei sich das Gewindeloch durch den Körper des Aktuators erstreckt, wobei der Körper ein zweites Loch definiert, das einen Stift aufnimmt, dessen Länge größer ist als die Breite des Körpers.

10. Prothese nach einem der vorstehenden Ansprüche, wobei eine Spitze des einziehbaren Elements einen Vorsprung aufweist, der konfiguriert ist, um den Aktuator im inneren Hohlraum zu halten.

11. Prothese nach Anspruch 1, wobei:
die Schaftkomponente dazu bemessen und konfiguriert ist, um in einem intramedullären Kanal aufgenommen zu werden, der in einem Knochen ausgebildet ist, wobei der Aktuator konfiguriert ist, um das einziehbare Element von der ersten Position in die zweite Position zu bewegen; und
wobei das erste Gegendrehmomentmerkmal konfiguriert ist, um einer Drehung der Schaftkomponente zu widerstehen, wenn der Aktuator betätigt wird.

12. Prothese nach Anspruch 11, wobei das einziehbare Element einen Vorsprung aufweist, der konfiguriert ist, um den Aktuator im inneren Hohlraum zu halten; oder wobei das erste Gegendrehmomentmerkmal eine Oberflächenstruktur am hinteren Ende der Schaftkomponente aufweist; oder wobei das erste Gegendrehmomentmerkmal einen Vorsprung aufweist, der sich vom Aktuator erstreckt und in einem durch die Schaftkomponente definierten Schlitz angeordnet ist; oder wobei das erste Gegendrehmomentmerkmal einen Vorsprung aufweist, der sich vom hinteren Ende der Schaftkomponente erstreckt; oder wobei das erste Gegendrehmomentmerkmal eine Aussparung aufweist, die durch das hintere Ende der Schaftkomponente definiert ist, wobei die Aussparung dazu bemessen und konfiguriert ist, um einen Vorsprung aufzunehmen, der sich von einer Oberfläche einer anderen Komponente der Prothese erstreckt; oder wobei das erste Gegendrehmomentmerkmal eine am hinteren Ende der Schaftkomponente ausgebildete Abflachung aufweist, wobei die Abflachung konfiguriert ist, um mit einer komplementären Oberfläche einer anderen Komponente der Prothese in Eingriff zu treten.

13. Verfahren nach Anspruch 11, wobei der Aktuator aufweist:
einen ersten Abschnitt;
einen zweiten Abschnitt, der einen Gewindeschaft aufweist, der sich durch ein Loch erstreckt, das durch das hintere Ende des Schafts definiert wird; und
einen dritten Abschnitt, der zwischen dem ersten Abschnitt und dem zweiten Abschnitt angeordnet ist, wobei der dritte Abschnitt eine Nockenoberfläche aufweist, wobei ein Sicherungselement üblicherweise entlang einer Länge des zweiten Abschnitts angeordnet ist und den Aktuator im inneren Hohlraum hält.

14. Prothese nach Anspruch 13, wobei das Sicherungselement einen Spaltring aufweist; oder wobei die Prothese ferner eine verformbare Unterlegscheibe umfasst, die zwischen dem hinteren Ende des Schafts und dem Sicherungselement angeordnet ist; oder wobei der erste Abschnitt des Aktuators einen Vorsprung aufweist, der in einem Schlitz aufgenommen ist, der neben dem einziehbaren Element angeordnet ist, wobei das hintere Ende der Schaftkomponente ein zweites Gegendrehmomentmerkmal (542) aufweist und wobei das zweite Gegendrehmomentmerkmal eine Oberflächenstruktur am hinteren Ende der Schaftkomponente aufweist.

15. Prothese nach Anspruch 14, wobei das zweite Gegendrehmomentmerkmal einen Vorsprung aufweist, der sich vom Aktuator erstreckt und in einem durch die Schaftkomponente definierten Schlitz angeordnet ist; oder wobei das zweite Gegendrehmomentmerkmal einen Vorsprung aufweist, der sich vom hinteren Ende der Schaftkomponente erstreckt; oder wobei das zweite Gegendrehmomentmerkmal eine Aussparung aufweist, die durch das hintere Ende der Schaftkomponente definiert ist, wobei die Aussparung dazu bemessen und konfiguriert ist, um einen Vorsprung aufzunehmen, der sich von einer Oberfläche einer anderen Komponente der Prothese erstreckt; oder wobei das zweite Gegendrehmomentmerkmal eine Abflachung am hinteren Ende der Schaftkomponente aufweist, wobei die Abflachung konfiguriert ist, um mit einer komplementären Oberfläche einer anderen Komponente der Prothese in Eingriff zu treten.

16. Prothese nach Anspruch 11, wobei der Aktuator einen Körper mit einem ersten und einem zweiten Abschnitt aufweist, wobei sich der zweite Abschnitt vom ersten Abschnitt aus verjüngt und eine Nockenoberfläche bereitstellt, wobei der Körper ein Loch definiert, das an einem Ende zumindest teilweise mit einem Gewinde versehen ist, wobei das erste Gegendrehmomentmerkmal optional einen Vorsprung aufweist, der sich vom ersten Abschnitt erstreckt und in einem Schlitz aufgenommen ist, der neben dem einziehbaren Element angeordnet ist.

17. Prothese nach Anspruch 16, wobei sich das durch den Körper des Aktuators definierte Loch durch den Körper erstreckt, wobei der Körper ein Querloch definiert, in dem ein Stift angeordnet ist, wobei der Stift eine Länge aufweist, die größer ist als die Breite des Körpers, sodass mindestens ein Abschnitt des Stifts in einem Schlitz aufgenommen wird, der neben dem einziehbaren Element angeordnet ist, wobei das erste Gegendrehmomentmerkmal eine Oberflächenstruktur am hinteren Ende der Schaftkomponente aufweist, oder wobei das erste Gegendrehmomentmerkmal einen Vorsprung aufweist, der sich vom hinteren Ende der Schaftkomponente erstreckt, oder wobei das erste Gegendrehmomentmerkmal eine durch das hintere Ende der Schaftkomponente definierte Aussparung aufweist, wobei die Aussparung dazu bemessen und konfiguriert ist, um einen Vorsprung aufzunehmen, der sich von einer Oberfläche einer anderen Komponente der Prothese erstreckt, oder wobei das erste Gegendrehmomentmerkmal eine am hinteren Ende der Schaftkomponente ausgebildete Abflachung aufweist, wobei die Abflachung konfiguriert ist, um mit einer komplementären Oberfläche einer anderen Komponente der Prothese in Eingriff zu treten.

## Revendications

1. Prothèse de cheville (600), comprenant :
un premier composant (620) définissant un trou et configuré pour être fixé à une surface articulaire d'articulation prothétique, dans laquelle le premier composant est un composant de plateau tibial ; et
un deuxième composant (610) configuré pour être couplé au premier composant,
dans laquelle le deuxième composant est un composant de tige de tibia, le deuxième composant comprenant une extrémité d'attaque (611) et une extrémité de fuite (612), le deuxième composant définissant une cavité interne (615) et comprenant un élément rétractable (630), l'élément rétractable étant configuré pour être déplacé d'une première position à une deuxième position de manière peropératoire ; et
un actionneur (617) configuré pour être reçu dans et se déplacer le long de la cavité interne du deuxième composant,
l'actionneur comprenant une première caractéristique de couple antagoniste (618) configurée pour mettre en prise le deuxième composant et pour résister à la rotation de l'actionneur, lorsque l'actionneur est mis en prise par un troisième composant.

2. Prothèse selon la revendication 1, dans laquelle le troisième composant comprend un écrou de base (625),
et dans laquelle l'actionneur comprend une tige filetée (617-S) qui est configurée pour être mise en prise par l'écrou de base.

3. Prothèse selon la revendication 2, dans laquelle l'actionneur comprend une surface de came (617-C) disposée de manière adjacente à la tige filetée, la surface de came étant configurée pour mettre en prise une surface interne (630-C) de l'élément rétractable, dans laquelle typiquement, la première caractéristique de couple antagoniste comprend une saillie s'étendant à partir d'une partie de tête de l'actionneur qui est disposée de manière adjacente à la surface de came, et dans laquelle la saillie est configurée pour être reçue dans une fente disposée de manière adjacente à l'élément rétractable.

4. Prothèse selon l'une quelconque des revendications 2 et 3, comprenant en outre une rondelle (850) dimensionnée et configurée pour être reçue entre le premier composant et le deuxième composant et pour recevoir la tige filetée, la rondelle étant configurée pour se déformer en réponse à une pression appliquée par le premier composant et le deuxième composant.

5. Prothèse selon la revendication 4, comprenant en outre un élément de fixation (960) configuré pour fixer l'actionneur à l'intérieur de la cavité interne, dans laquelle typiquement, l'élément de fixation comprend une bague fendue dimensionnée et configurée pour être disposée sur la tige filetée de l'actionneur.

6. Prothèse selon l'une quelconque des revendications précédentes, dans laquelle l'extrémité de fuite du deuxième composant comprend une deuxième caractéristique de couple antagoniste (542), la deuxième caractéristique de couple antagoniste étant configurée pour mettre en prise le premier composant et pour résister à la rotation du deuxième composant, lorsque l'actionneur est mis en prise par le troisième composant.

7. Prothèse selon la revendication 6, dans laquelle la deuxième caractéristique de couple antagoniste comprend une texturation de surface ; ou bien dans laquelle la deuxième caractéristique de couple antagoniste comprend une saillie qui est configurée pour être reçue dans un évidement correspondant défini par le premier composant ;
ou bien dans laquelle la deuxième caractéristique de couple antagoniste comprend une surface plate sur l'extrémité de fuite du deuxième composant qui est configurée pour mettre en prise une surface correspondante sur le premier composant ;
ou bien dans laquelle la deuxième caractéristique de couple antagoniste comprend un évidement qui est configuré pour recevoir une saillie s'étendant à partir d'une surface du premier composant.

8. Prothèse selon l'une quelconque des revendications précédentes, dans laquelle le troisième composant comprend un boulon fileté, et dans laquelle l'actionneur comprend un corps ayant une surface de came et une partie de tête, le corps définissant un trou fileté au niveau d'une extrémité, qui est configuré pour recevoir, au moins partiellement, le boulon fileté.

9. Prothèse selon la revendication 8, dans laquelle la première caractéristique de couple antagoniste comprend une saillie s'étendant à partir de la partie de tête, la saillie étant dimensionnée et configurée pour être reçue dans une fente disposée de manière adjacente à l'élément rétractable ;
ou bien dans laquelle le trou fileté est un trou borgne ; ou bien dans laquelle le trou fileté s'étend à travers le corps de l'actionneur,
le corps définissant un deuxième trou qui reçoit une broche ayant une longueur qui est supérieure à une largeur du corps.

10. Prothèse selon l'une quelconque des revendications précédentes, dans laquelle une pointe de l'élément rétractable comprend une protubérance configurée pour retenir l'actionneur à l'intérieur de la cavité interne.

11. Prothèse selon la revendication 1, dans laquelle :
le composant de tige est dimensionné et configuré pour être reçu dans un canal intramédullaire formé dans un os, l'actionneur étant configuré pour déplacer l'élément rétractable de la première position à la deuxième position ; et
dans laquelle la première caractéristique de couple antagoniste est configurée pour résister à la rotation du composant de tige lorsque l'actionneur est actionné.

12. Prothèse selon la revendication 11, dans laquelle l'élément rétractable comprend une protubérance configurée pour retenir l'actionneur à l'intérieur de la cavité interne ;
ou bien dans laquelle la première caractéristique de couple antagoniste comprend une texture de surface au niveau de l'extrémité de fuite du composant de tige ; ou bien dans laquelle la première caractéristique de couple antagoniste comprend une saillie s'étendant à partir de l'actionneur et disposée à l'intérieur d'une fente définie par le composant de tige ; ou bien dans laquelle la première caractéristique de couple antagoniste comprend une saillie s'étendant à partir de l'extrémité de fuite du composant de tige ; ou bien dans laquelle la première caractéristique de couple antagoniste comprend un évidement défini par l'extrémité de fuite du composant de tige, l'évidement étant dimensionné et configuré pour recevoir une protubérance qui s'étend à partir d'une surface d'un autre composant de la prothèse ;
ou bien dans laquelle la première caractéristique de couple antagoniste comprend une facette formée au niveau de l'extrémité de fuite du composant de tige, la facette étant configurée pour être mise en prise par une surface complémentaire d'un autre composant de la prothèse.

13. Prothèse selon la revendication 11, dans laquelle l'actionneur comprend une première partie ;
une deuxième partie comprenant un arbre fileté qui s'étend à travers un trou défini par l'extrémité de fuite de la tige ; et
une troisième partie disposée entre la première partie et la deuxième partie, la troisième partie comprenant une surface de came, dans laquelle un élément de fixation est typiquement disposé le long d'une longueur de la deuxième partie et retient l'actionneur à l'intérieur de la cavité interne.

14. Prothèse selon la revendication 13, dans laquelle l'élément de fixation comprend une bague fendue ; ou bien dans laquelle la prothèse comprend en outre une rondelle déformable disposée entre l'extrémité de fuite de la tige et l'élément de fixation ; ou bien dans laquelle la première partie de l'actionneur comprend une protubérance qui est reçue à l'intérieur d'une fente disposée de manière adjacente à l'élément rétractable,
dans laquelle l'extrémité de fuite du composant de tige comprend une deuxième caractéristique de couple antagoniste (542), et dans laquelle la deuxième caractéristique de couple antagoniste comprend une texture de surface au niveau de l'extrémité de fuite du composant de tige.

15. Prothèse selon la revendication 14, dans laquelle la deuxième caractéristique de couple antagoniste comprend une saillie s'étendant à partir de l'actionneur et disposée à l'intérieur d'une fente définie par le composant de tige ; ou bien dans laquelle la deuxième caractéristique de couple antagoniste comprend une saillie s'étendant à partir de l'extrémité de fuite du composant de tige ; ou bien dans laquelle la deuxième caractéristique de couple antagoniste comprend un évidement défini par l'extrémité de fuite du composant de tige, l'évidement étant dimensionné et configuré pour recevoir une protubérance qui s'étend à partir d'une surface d'un autre composant de la prothèse ;
ou bien dans laquelle la deuxième caractéristique de couple antagoniste comprend une facette formée au niveau de l'extrémité de fuite du composant de tige, la facette étant configurée pour être mise en prise par une surface complémentaire d'un autre composant de la prothèse.

16. Prothèse selon la revendication 11, dans laquelle l'actionneur comprend un corps ayant une première partie et une deuxième partie, la deuxième partie se rétrécissant progressivement à partir de la première partie et fournissant une surface de came, le corps définissant un trou qui est au moins partiellement fileté au niveau d'une extrémité, dans laquelle facultativement la première caractéristique de couple antagoniste comprend une saillie s'étendant à partir de la première partie et étant reçue à l'intérieur d'une fente disposée de manière adjacente à l'élément rétractable.

17. Prothèse selon la revendication 16, dans laquelle le trou défini par le corps de l'actionneur s'étend à travers le corps, le corps définissant un trou transversal dans lequel est disposée une broche, la broche ayant une longueur qui est supérieure à une largeur du corps, de sorte qu'au moins une partie de la broche est reçue à l'intérieur d'une fente disposée de manière adjacente à l'élément rétractable,
dans laquelle la première caractéristique de couple antagoniste comprend une texture de surface au niveau de l'extrémité de fuite du composant de tige, ou bien dans laquelle la première caractéristique de couple antagoniste comprend une saillie s'étendant à partir de l'extrémité de fuite du composant de tige, ou bien dans laquelle la première caractéristique de couple antagoniste comprend un évidement défini par l'extrémité de fuite du composant de tige, l'évidement étant dimensionné et configuré pour recevoir une protubérance qui s'étend à partir d'une surface d'un autre composant de la prothèse,
ou bien dans laquelle la première caractéristique de couple antagoniste comprend une facette formée au niveau de l'extrémité de fuite du composant de tige, la facette étant configurée pour être mise en prise par une surface complémentaire d'un autre composant de la prothèse.
